# EUROPÄISCHE PATENTSCHRIFT

(11) **EP 0 320 455 B1**
(45) Veröffentlichungstag und Bekanntmachung des Hinweises auf die Patenterteilung: **09.06.1993**
(21) Anmeldenummer: 88810830.5
(22) Anmeldetag: 02.12.1988
(51) Int. Cl.: C07F 9/38, C07F 9/58, C07F 9/547, A61K 31/66

(54) **Araliphatylaminoalkandiphosphonsäuren**
Araliphatyl aminoalcane diphosphonic acids
Acides aminoalcanediphosphoniques araliphatiques

(30) Priorität: 11.12.1987 CH 4847/87
(43) Veröffentlichungstag der Anmeldung: 14.06.1989
(73) Patentinhaber: CIBA-GEIGY AG, 4002 Basel (CH)
(72) Erfinder: Jaeggi, Knut A., Dr., CH-4054 Basel (CH)

(56) Entgegenhaltungen:
- EP-A- 0 186 405
- EP-A- 0 252 504
- DE-A- 3 623 397

## Beschreibung

Die Erfindung betrifft aromatisch substituierte Alkylaminoalkandiphosphonsäuren der Formel 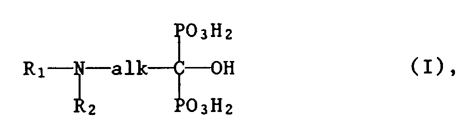
worin R₁ einen durch einen unsubstituierten oder durch Niederalkyl, Niederalkoxy, Niederalkylthio und/oder Halogen substituierten 6-gliedrigen monocyclischen oder aus 5- oder 6-gliedrigen Ringen aufgebauten bicyclischen Aryl-bzw. als Heteroatom(e) 1 oder 2 N-Atome, 1 O- oder S-Atom, 1 N- und 1 O-Atom oder 1 N- und 1 S-Atom aufweisenden Heteroarylrest mono- oder disubstituierten Niederalkyl- oder Niederalkenylrest bedeutet, R₂ Wasserstoff, Niederalkyl oder Niederalkenyl darstellt und alk für Niederalkylen steht, mit der Massgabe, dass R₂ Wasserstoff oder C₁-C₃-Alkyl ist und R₁ im aliphatischen Teil 2 oder 3 C-Atome aufweist, wenn R₁ durch unsubstituiertes Phenyl monosubstituiert ist, und ihre Salze; wobei mit "Nieder" bezeichnete Reste höchstens 7 C-Atome aufweisen, Verfahren zur Herstellung der erfindungsgemässen Verbindungen, diese enthaltende pharmazeutische Präparate und ihre Verwendung als Arzneimittelwirkstoffe.

In EP-A-186 405 sind bereits Verbindungen der Formel I, worin R₁ durch gegebenenfalls substituiertes Pyridyl, Pyridazinyl, Pyrimidinyl oder Pyrazinyl substituiertes Niederalkyl, R₂ Wasserstoff oder einen aliphatischen Rest und alk Niederalkylen bedeutet, zur Behandlung von Calciumstoffwechselstörungen vorgeschlagen worden. Ferner sind in EP-A-252 504 (Zwischenliteratur) Verbindungen der Formel I, in denen (a) R₁ (unsubstituiertes) Benzyl und R₂ C₁-C₉-Alkyl oder C₂-C₉-Alkenyl, (b) R₁ durch (unsubstituiertes) Phenyl substituiertes C₁-C₉-Alkyl oder C₂-C₉-Alkenyl und R₂ C₄-C₁₈-Alkyl oder C₄-C₁₈-Alkenyl, und (c) R₁ durch (unsubstituiertes) Phenyl substituiertes C₄-C₁₈-Alkyl oder C₄-C₁₈-Alkenyl und R₂ C₁-C₉-Alkyl oder C₂-C₉-Alkenyl ist, mit den Calciumstoffwechsel regulierender Wirkung beschrieben. In DE-A-3 623 397 sind Verbindungen der Formel I, in denen (a) R₁ (unsubstituiertes) Benzyl und R₂ C₁-C₉-Alkyl oder C₂-C₉-Alkenyl, (b) R₁ durch (unsubstituiertes) Phenyl substituiertes C₁-C₉-Alkyl oder C₂-C₉-Alkenyl und R₂ C₃-C₁₈-Alkyl oder C₃-C₁₈-Alkenyl, und (c) R₁ durch (unsubstituiertes) Phenyl substituiertes C₃-C₁₈-Alkyl oder C₃-C₁₈-Alkenyl und R₂ C₁-C₉-Alkyl oder C₂-C₉-Alkenyl ist, mit den Calciumstoffwechsel regulierender Wirkung beschrieben.

Aromatische bzw. heteroaromatische Substituenten von Niederalkyl- oder Niederalkylenresten sind beispielsweise unsubstituierte oder, insbesondere durch Niederalkyl, Niederalkoxy, Niederalkylthio und/oder Halogen, ein- oder mehrfach, vorzugsweise einfach oder in zweiter Linie zweifach, substituierte 5- oder 6-gliedrige monocyclische oder aus 5-oder 6-gliedrigen Ringen aufgebaute bicyclische Aryl- bzw. als Heteroatom(e) 1 oder 2 N-Atome, 1 O- oder S-Atom, 1 N- und 1 O-Atom oder 1 N-und 1 S-Atom aufweisende Heteroarylreste, wie Phenyl oder in zweiter Linie Naphthyl bzw. Pyrryl, Thienyl, Furyl, Pyridyl, Imidazolyl, Pyrimidinyl oder Chinolinyl, insbesondere Phenyl, Thienyl, Pyridyl oder Imidazolyl. Der Rest R₁ kann durch einen oder mehr als einen, beispielsweise einen oder zwei, gleiche oder verschiedene solcher Substituenten R₃ substituiert sein. Diese sind vorzugsweise über ein C-Atom gebunden, können aber auch über das gegebenenfalls vorhandene zusätzliche N-Atom gebunden sein. Weist R₁ mehrere solcher Substituenten auf, sind vorzugsweise zwei derselben an das gleiche C-Atom des aliphatischen Restes gebunden. Als Reste R₁ kommen vorzugsweise solche der Formel 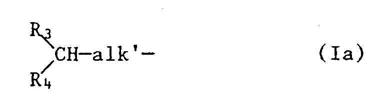
in Betracht, worin R₃ einen aromatischen Rest R_{A}, R₄ Wassestoff oder einen aromatischen Rest R_{B} und alk' Niederalkylen bedeutet, wobei R_{A} und R_{B} jeweils eine der für R angegebenen Bedeutungen haben und die Summe der Kohlenstoffatome von alk' vorzugsweise nicht mehr als 6 beträgt.

Nachstehend sind unter niederen Resten und Verbindungen beispielsweise solche zu verstehen, die bis und mit 7, insbesondere bis und mit 4, C-Atome aufweisen. Ferner haben die Allgemeinbegriffe beispielsweise folgende Bedeutungen:
Niederalkyl ist beispielsweise C₁-C₄-Alkyl, wie Methyl, Aethyl, Propyl, Isopropyl oder Butyl, ferner Iso-, Sekundär- oder Tertiärbutyl, kann aber auch eine C₅-C₇-Alkyl-, wie Pentyl-, Hexyl- oder Heptylgruppe bedeuten.

Niederalkoxy ist beispielsweise C₁-C₄-Alkoxy, wie Methoxy, Aethoxy, Propyloxy, Isopropyloxy oder Butyloxy, ferner Iso-, Sekundär- oder Tertiärbutyloxy.

Niederalkylthio ist beispielsweise C₁-C₄-Alkylthio, wie Methylthio, Aethylthio, Propylthio oder Butylthio, ferner Iso-, Sekundär- oder Tertiärbutylthio.

Halogen ist beispielsweise Halogen der Atomnummer bis und mit 35, wie Chlor oder Fluor, ferner Brom.

Niederalkenyl ist beispielsweise C₂-C₄-Alkenyl, wie Vinyl, Allyl oder Buten-2-yl, kann aber auch eine C₅-C₇-Alkenyl-, wie Pentenyl-, Hexenyl- oder Heptenylgruppe sein.

Niederalkylen alk ist beispielsweise C₂-C₄-Alkylen, vor allem geradkettiges C₂-C₄-Alkylen, wie α,ω-C₂-C₄-Alkylen, z.B.Aethylen, 1,3-Propylen oder in zweiter Linie 1,4-Butylen.

Niederalkylen alk' ist beispielsweise C₂-C₆-Alkylen, vor allem geradkettiges C₂-C₆-Alkylen, wie α,ω-C₂-C₆-Alkylen, z.B. Aethylen, 1,3-Propylen, 1,4-Butylen, 1,5-Pentylen oder 1,6-Hexylen, kann aber auch 1,2-Propylen, 1,2- oder 1,3-Butylen oder 1,4-Pentylen sein.

Salze von Verbindungen der Formel I sind insbesondere deren innere Salze oder Salze mit pharmazeutisch verwendbaren Basen, wie nicht-toxische, von Metallen oder Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Aethyl-, Dimethyl- oder Diäthylamin, Mono-, Di- oder Tri-(hydroxyniederalkyl)-aminen, wie Aethanol-, Diäthanol- oder Triäthanolamin, Tris(hydroxymethyl)-amino-methan oder 2-Hydroxytertiärbutylamin, oder N-(Hydroxyniederalkyl)-N,N-diniederalkylaminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. mit Tetrabutylammoniumhydroxid. Umfasst sind sowohl vollständige als auch partielle Salze, d.h. Salze mit 1,2,3 oder 4, vorzugsweise 2, Aequivalenten Base pro Mol der Säure der Formel I.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) -Rattenmodell anhand der durch Vitamin D₃ ausgelösten experimentellen Hypercalcämie nach Gabe von Dosen von etwa 0,001 bis 1.0 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,01 bis 1,0 mg/kg s.c. eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Die Erfindung betrifft bevorzugt Verbindungen der Formel I, worin R₁ einen durch einen unsubstituierten oder durch Niederalkyl, Niederalkoxy, Niederalkylthio und/oder Halogen substituierten 6-gliedrigen monocyclischen oder aus 5- und/oder 6-gliedrigen Ringen aufgebauten bicyclischen Arylrest mono- oder disubstituierten oder durch einen 5-oder 6-gliedrigen, als Heteroatome 1 O- oder S-Atom oder 1 oder 2 N-Atome aufweisenden monocyclischen Heteroarylrest monosubstituierten Niederalkylrest bedeutet, R₂ Wasserstoff oder einen Niederalkylrest darstellt und alk für Niederalkylen steht, mit der Massgabe, dass R₂ Wasserstoff ist, wenn R₁ einen unsubstituierten Benzyl- oder Phenyl-C₃-C₇-alkylrest bedeutet, bzw. R₂ Wasserstoff oder Niederalkyl mit 1 oder 2 C-Atomen ist, wenn R₁ einen Phenyläthylrest bedeutet, und ihre Salze.

Die Erfindung betrifft insbesondere Verbindungen der Formel I, worin R₁ einen Rest der Formel 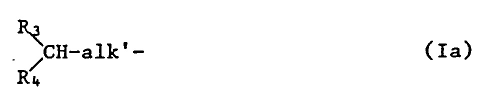
bedeutet, in der R₃ einen aromatischen Rest R_{A}, R₄ Wasserstoff oder einen aromatischen Rest R_{B} und alk' Niederalkylen bedeutet, wobei R_{A} bzw.R_{B} jeweils einen unsubstituierten oder durch Niederalkyl, Niederalkoxy, Niederalkylthio und/oder Halogen substituierten 5- oder 6-gliedrigen monocyclischen oder aus 5- oder 6-gliedrigen Ringen aufgebauten bicyclischen Aryl- bzw. als Heteroatom(e) 1 oder 2 N-Atome, 1 O- oder S-Atom, 1 N- und 1 O-Atom oder 1 N- und 1 S-Atom aufweisenden Heteroarylrest darstellt, R₂ Wasserstoff, Niederalkyl oder Niederalkenyl bedeutet, und alk für Niederalkylen steht, und ihre Salze.

Die Erfindung betrifft vor allem einerseits Verbindungen der Formel I, worin R₁ einen Rest der Formel 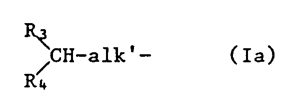
bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest darstellt, alk' für C₁-C₅-Alkylen steht, R₂ für Wasserstoff steht und alk geradkettiges C₂-C₄-Alkylen darstellt, und ihre Salze.

Die Erfindung betrifft vor allem andererseits Verbindungen der Formel I, worin R₁ einen Rest der Formel 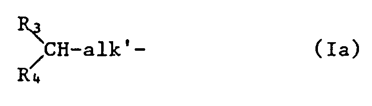
bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₅-Alkoxy, C₁-C₄-Alkylthio und/oder Halogen der Atomnummer bis und mit 35 mono- oder disubstituierten Phenylrest darstellt, alk' für geradkettiges C₁-C₅-Alkylen steht, R₂ C₁-C₄-Alkyl bzw. C₂-C₄-Alkenyl darstellt und alk geradkettiges C₂-C₄-Alkylen darstellt, mit der Massgabe, dass alk' Methylen und R₂ C₁-C₂-Alkyl ist, wenn R₃ unsubstituiertes Phenyl und R₄ Wasserstoff bedeutet, und ihre Salze.

Die Erfindung betrifft vorzugsweise Verbindungen der Formel I, worin R₁ einen Rest der Formel 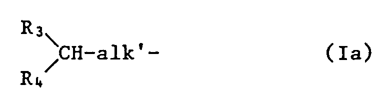
darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ für Wasserstoff steht, alk' geradkettiges, terminal gebundenes C₂-C₄-Alkylen bedeutet, R₂ Wasserstoff bedeutet oder, sofern alk' C₁-C₂-Alkylen bedeutet, für C₁-C₃-Alkyl steht und alk C₂-C₃-Alkylen darstellt, und ihre Salze.

Die Erfindung betrifft ganz bevorzugt Verbindungen der Formel I, worin R₁ unsubstituiertes oder im Phenylteil durch C₁-C₄-Alkyl, C₁-C₄-Alkoxy und/oder Halogen der Atomnummer bis und mit 35 mono- oder disubstituiertes Mono- oder Diphenyl-C₂-C₆-alkyl oder Imidazolyl-, Thienyl- oder Pyridyl-C₂-C₆-alkyl bedeutet, R₂ für Wasserstoff oder C₁-C₄-Alkyl steht und alk C₂-C₃-Alkylen darstellt, mit der Massgabe, dass R₂ Wasserstoff ist, wenn R₁ unsubstituiertes Phenyl-C₃-C₄-alkyl bedeutet, bzw. R₂ Wasserstoff oder C₁-C₂-Alkyl ist, wenn R₁ einen unsubstituierten Phenyläthylrest darstellt, und ihre Salze.

Die Erfindung betrifft in allererster Linie Verbindungen der Formel I, worin R₁ einen Rest der Formel 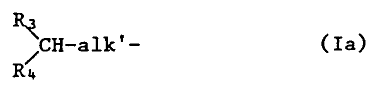
darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ Wasserstoff ist, alk' geradkettiges, terminal gebundenes C₂-C₄-Alkylen bedeutet, R₂ Wasserstoff bedeutet und alk C₂-C₃-Alkylen darstellt, und ihre Salze.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man
a) in einer Verbindung der Formel 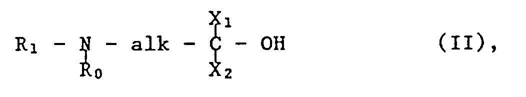 worin R₀ einen Rest R₂ oder eine Aminoschutzgruppe darstellt und X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln 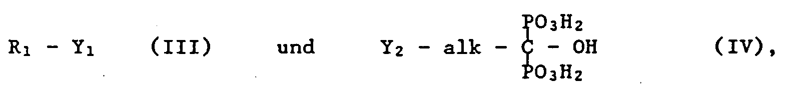 worin einer der Reste Y₁ und Y₂ eine reaktionsfähige veresterte Hydroxygruppe und der andere eine Gruppe der Formel -N(R₀)-H bedeutet, in der R₀ einen Rest R₂ oder eine Aminoschutzgruppe darstellt, oder Salze davon, miteinander umsetzt, oder
c) eine Verbindung der Formel 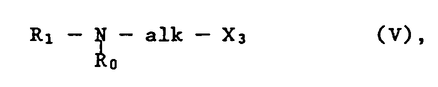 worin R₀ einen Rest R₂ oder eine Aminoschutzgruppe darstellt und X₃ Carboxy, Carbamyl oder Cyano, insbesondere Carboxy oder Cyano, bedeutet, mit phosphoriger Säure und Phosphortrichlorid umsetzt, das Primärprodukt hydrolysiert und in einem ausgehend von Verbindungen der Formel V, worin X₃ Cyano oder Carbamyl ist, erhaltenen Zwischenprodukt der Formel 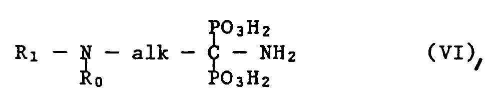 bzw. einem Salz davon, die Aminogruppe durch Behandlung mit salpetriger Säure durch Hydroxy ersetzt; jeweils die Aminoschutzgruppe, sofern vorhanden, abspaltet und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Als Aminoschutzgruppe R₀ kommen z.B. α-Arylniederalkyl, wie Benzyl oder p-Methoxybenzyl, α,α,α-Triarylniederalkyl, wie Trityl, oder Triniederalkyl-, wie Trimethylsilyl in Betracht. α-Aryl- und α,α,α-Tri-arylniederalkyl kann hydrogenolytisch, Triniederalkylsilyl und α,α,α-Tri-arylniederalkyl hydrolytisch leicht wieder entfernt werden. Die hydrogenolytische Abspaltung von α-Aryl- bzw. α,α,α-Tricosylniederalkyl R₀ erfolgt insbesondere durch Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalem Druck- und Temperaturbedingungen, z.B. bei etwa 20°C bis 30°C und etwa 0,95 bis etwa 1,3 bar.

Gemäss der Verfahrensvariante a) in Phosphono zu überführende funktionell abgewandelte Phosphonogruppen liegen beispielsweise in einer Esterform, insbesondere einer Diesterform der Formel -P(=O)(OR)₂ (IIa), vor, worin OR veräthertes Hydroxy, beispielsweise Niederalkoxy, Niederalkanoyloxyniederalkoxy, wie C₂-C₇-Alkanoyloxy-C₁-C₄-alkoxy, z.B. Acetyl- oder Pivaloyloxymethoxy, oder eine gegebenenfalls durch Niederalkyl, Niederalkoxy, Halogen, Trifluormethyl und/oder Hydroxy substituierte Phenyloxy- oder α-Phenylniederalkoxygruppe oder Silyloxy, wie Triniederalkylsilyloxy, bedeutet.

Die Ueberführung von funktionell abgewandelten in freie Phosphonogruppen erfolgt in üblicher Weise, wie durch Hydrolyse, beispielsweise in Gegenwart einer Mineralsäure, wie Salz- oder Schwefelsäure, bei etwa 80°C bis etwa 110°C, z.B. in der Siedehitze, oder durch Umsetzung mit einem Triniederalkyl-halogensilan, z.B. mit Trimethyl-chlorsilan oder insbesondere Trimethyl-jodsilan oder Trimethyl-bromsilan, vorzugsweise in Methylenchlorid im Temperaturbereich von etwa 0° bis etwa 40°C, und anschliessende Behandlung mit Wasser. α-Phenylniederalkylester können ferner durch Hydrogenolyse, beispielsweise Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalen Druck- und Temperaturbedingungen in Verbindungen der Formel I überführt werden.

Die Ausgangsstoffe der Formel II können beispielsweise hergestellt werden, indem man eine Verbindung der Formel 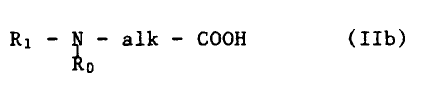
oder vorzugsweise das Anhydrid oder Säurechlorid derselben mit einem entsprechenden Phosphorigsäuretriester der Formel P(OR)₃ (IIc), beispielsweise bei 0°C bis etwa 60°C, zu einer Verbindung der Formel 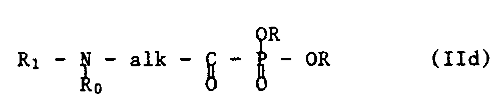
und diese mit einem Phosphorigsäurediester der Formel H-P(=O)(OR)₂ (IIe) bzw. P(OH)(OR)₂ (IIf) in Gegenwart eines Diniederalkylamins, z.B. von Diäthylamin, oder eines Alkalimetallniederalkanolats, z.B. Natriummethylat, zur entsprechenden Verbindung der Formel 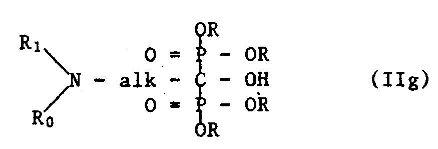
weiterumsetzt.

Ausgangsstoffe der Formel IIb können, soweit sie nicht bekannt sind, beispielsweise hergestellt werden, indem man eine entsprechende Verbindung der Formel

R₁-N(R₀)-H (IIh),

worin R₀ eine Gruppe R₂ oder eine Aminoschutzgruppe bedeutet, mit einer Verbindung der Formel

Y - alk - COOR (IIi),

worin Y Halogen, wie Brom ist, oder zur Herstellung von Verbindungen IIb, worin alk 1,2-Niederalkylen, z.B. Aethylen, bedeutet, einer Verbindung der Formel

alk₀ - COOR (IIj)

worin alk₀ Niederalk-1-enyl bedeutet, umsetzt, jeweils den erhaltenen Ester zur Säure hydrolysiert, diese, z.B. mittels Phosphorpentachlorid, anhydridisiert bzw. chloriert und gewünschtenfalls die Aminoschutzgruppe, sofern vorhanden, abspaltet.

Gemäss der Verfahrensvariante b) zu verwendende reaktive Ester (III) bzw. (IV) weisen als reaktionsfähige veresterte Hydroxygruppe beispielsweise ein Halogen-, wie Chlor-, Brom- oder Jodatom oder eine Sulfonyloxygruppe, wie Alkan- oder gegebenenfalls substituiertes Benzolsulfonyloxy, z.B. Methan- oder p-Toluolsulfonyloxy, auf.

Die Umsetzung mit den genannten reaktiven Estern erfolgt beispielsweise in Gegenwart einer Base, wie eines Alkalimetall- oder Erdalkalimetallhydroxides, z.B. von Natriumhydroxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Aethers, z.B. von Isopropanol, Methyläthylketon, Dioxan oder Tetrahydrofuran.

Die Ausgangsstoffe der Formel IV können beispielsweise hergestellt werden, indem man eine Verbindung der Formel

Y₂ - alk - COOH (IVa)

in üblicher Weise, beispielsweise in Chlorbenzol, mit phosphoriger Säure und Phosphortrichlorid bzw. mit Phosphorsäure und einem Ueberschuss an Phosphortribromid umsetzt und anschliessend hydrolytisch aufarbeitet.

Die Umsetzung von Verbindungen der Formel V mit phosphoriger Säure und Phosphortrichlorid gemäss der Verfahrensvariante c) erfolgt in üblicher Weise, wobei die Phophorigsäurekomponente vorzugsweise durch Reaktion überschüssigen Phosphortrichlorides mit wasserhaltiger Phosphorsäure, z.B. mit handelsüblicher etwa 75%-iger bis etwa 95%-iger, vorzugsweise etwa 85%-iger Phosphorsäure, in situ gebildet wird. Die Umsetzung wird vorteilhaft unter Erwärmen, z.B. auf etwa 70° bis etwa 120°C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel VI mit salpetriger Säure erfolgt in üblicher Weise unter Freisetzung derselben in wässriger Lösung aus einem ihrer Salze, z.B. aus Natriumnitrit, durch Säurebehandlung, z.B. Einwirkung von Salzsäure, wobei intermediär ein entsprechendes, instabiles Diazoniumsalz, z.B. -chlorid, gebildet wird, das unter Einführung der α-Hydroxygruppe Stickstoff abspaltet.

Die Ausgangsstoffe der Formel V können, soweit sie nicht bekannt sind, beispielsweise hergestellt werden, indem man eine entsprechende Verbindung der Formel

R₁―N(R₀)―H (IIh),

worin R₀ eine Gruppe R₂ oder eine Aminoschutzgruppe bedeutet, mit einer Verbindung der Formel

Y - alk - X₃ (IIi),

worin Y Halogen, wie Brom ist, oder zur Herstellung von Verbindungen der Formel V, worin alk 1,2-Niederalkylen, z.B. Aethylen, bedeutet, mit einer Verbindung der Formel

alk₀ - X₃ (IIf)

worin alk₀ einen Niederalk-1-enylrest bedeutet, umsetzt, jeweils die Aminoschutzgruppe, sofern vorhanden, abspaltet und gewünschtenfalls jeweils das erhaltene Primärprodukt zur Säure hydrolysiert.

Verfahrensgemäss oder nach einem anderen an sich bekannten Verfahren erhaltene Verbindungen der Formel I können in an sich bekannter Weise in andere Verbindungen der Formel I überführt werden.

So kann man in einer erhaltenen Verbindung der Formel I, worin R₂ Wasserstoff bedeutet, diesen durch Umsetzung mit einem Niederalkanal unter reduzierenden Bedingungen, beispielsweise mit Formaldehyd und Ameisensäure, oder in zweiter Linie mit einem reaktionsfähigen Ester eines Niederalkanols bzw. Niederalkenols in üblicher Weise, vorzugsweise in Gegenwart eines basischen Kondensationsmittels, wie eines Alkalimetallniederalkanolates, durch einen Niederalkyl-bzw. Niederalkenylrest R₂ ersetzen.

Weiterhin kann man in R₁ und/oder R₂ vorhandene nicht-aromatische Doppelbindungen in üblicher Weise durch Hydrogenolyse, beispielsweise Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalen Druck- und Temperaturbedingungen, zu Einfachbindungen reduzieren.

Ferner kann man den bzw. die aromatischen Substituenten von R₁ substituieren, beispielsweise durch Umsetzung mit einem üblichen Kernhalogenierungsmittel, z.B. mit Chlor oder Brom in Gegenwart einer Lewissäure, wie Eisen-III-trichlorid, Halogen einführen.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I einschliesslich ihrer inneren Salze der Formel I können durch partielle oder vollständige Neutralisation mit einer der eingangs genannten Basen in Basesalze überführt werden. In analoger Weise kann man auch Säureadditionssalze in die entsprechenden freien Verbindungen deren innerer Salze überführen.

Umgekehrt kann man erhaltene freie Verbindungen der Formel I durch Behandlung mit einer Protonensäuren in Säureadditionssalze überführen.

Erhaltene Salze können in an sich bekannter Weise in andere Salze mit geringerem Kationenanteil (partielle Salze) oder in die freien Verbindungen umgewandelt werden, z.B. durch Behandeln mit einem sauren Reagens, wie einer Mineralsäure. Erhaltene freie Verbindungen können durch Behandlung mit einer Base, z.B. Alkalilauge in Salze und/oder erhaltene Salze mit geringerem Kationenanteil (partielle Salze) auf die gleiche Weise in solche mit höherem Kationenanteil, z.B. vollständige Salze, umgewandelt werden.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten.

Die neuen pharmazeutischen Präparate enthalten z.B. von etwa 10 % bis etwa 80 %, vorzugseise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragéee-Kernen verarbeitet.

Geeignete Trägerstoffe sind insbesondere Füllstoffe, wie Zucker, z.B. Lactose, Saccharose, Mannit oder Sorbit und Cellulosepräparate ferner Bindemittel, wie Stärkekleister unter Verwendung z.B. von Mais-, Weizen-, Reis- oder Kartoffelstärke, Gelatine, Tragaganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthyleglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die vorliegende Erfindung betrifft ebenfalls die Verwendung der Verbindungen der Formeln I und ihrer Salze, vorzugsweise zur Behandlung von auf Störungen des Calciumstoffwechsels zurückzuführenden Erkrankungen, z.B. des rheumatischen Formenkreises, und besonders von Osteoporosen.

Dosierungen unter 0,01 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch in hypertonischer Lösung subkutan, intramuskulär oder intravenös appliziert werden. Die bevorzugten Tagesdosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg, beispielsweise von etwa 0,013 bis 0,67 mg/kg.

Die Dosierung der verwendeten Verbindungen ist jedoch variabel und hängt von den jeweiligen Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg und kann bis zu 4-mal täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

### Beispiel 1:

(0,1 Mol) 3-[N-(3-Phenylpropyl)-N-methyl-amino]-propionsäure-hydrochlorid werden mit 13,4 ml 85%-iger Phosphorsäure und 50 ml Chlorbenzol unter Rühren und Rückfluss auf 100° erhitzt. Dann werden bei 100° 27 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende zähe Masse wird mit 100 ml 9-n Chlorwasserstoffsäure 3 Stunden unter Rühren und Rückfluss zum Sieden erhitzt. Man filtriert heiss unter Kohlezusatz und verdünnt das Filtrat mit Aceton, wobei sich die 3-[N-(3-Phenylpropyl)-N-methyl-amino]-1-hydroxy-propan-1,1-diphosphonsäure kristallin abscheidet; Smp. 219° (Zers.).

Das als Ausgangsmaterial dienende 3-[N-(3-Phenylpropyl)-N-methyl-amino]-propionsäure-hydrochlorid kann folgendermassen hergestellt werden:
(0,15 Mol) N-(3-Phenylpropyl)-N-methyl-amin werden in 50 ml Diäthyläther vorgelegt und unter Rühren allmählich mit 15,1 g Acrylsäureäthylester versetzt. Es bildet sich unter leichtem Anstieg der Temperatur eine klare Lösung. Nach Stehen über Nacht bei Raumtemperatur wird der Aether abdestilliert. Das zurückbleibende Oel stellt den rohen 3-[N-(3-Phenylpropyl)-N-methyl-amino]-propionsäureäthylester dar.

Der erhaltene Ester wird mit 600 ml 4-n Chlorwasserstoffsäure 24 Stunden zum Rückfluss erhitzt. Dann wird unter vermindertem Druck völlig eingedampft und der kristalline Rückstand mit Aceton verrieben. Nach Abnutschen, Waschen und Trocknen der Kristalle erhält man das 3-[N-(3-Phenylpropyl)-N-methyl-amino]-propionsäure-hydrochlorid.

### Beispiel 2:

Analog wie im Beispiel 1 beschrieben erhält man ausgehend von bzw. über
3-(3-Phenylpropylamino)-propionsäure-hydrochlorid (Smp. 219°, Zers.);
3-(3-Phenylprop-2-ylamino)-propionsäure-hydrochlorid (Smp. 126-127°);
3-[N-(3-Phenylpropyl)-N-äthyl-amino]-propionsäure-hydrochlorid, Oel, 3-(4-Phenylbutylamino)propionsäure hydrochlorid, Smp. 135-137°; und
3-[3-(Pyrid-3-yl)propylamino]-propionsäure-hydrochlorid die 3-(3-Phenylpropylamino)-1-hydroxy-propan-1,1-diphosphonsäure, Smp. 219° (Zers.);
3-(3-Phenylprop-2-ylamino)-1-hydroxy-propan-1,1-diphosphonsäure, Smp. 208-210° (Zers.);
3-[N-(3-Phenylpropyl)-N-äthyl-amino]-1-hydroxy-propan-1,1-diphosphonsäure, Smp. 195-197° (Zers.);
3-(4-Phenylbutylamino)-1-hydroxy-propan-1,1-diphosphonsäure, Smp 191-193° (Zers.), und
3-[3-(Pyrid-3-yl)propylamino]-1-hydroxy-propan-1,1-diphosphonsäure.

### Beispiel 3:

9,3 g (26,3 mMol) 3-(3-Phenylpropylamino)-1-hydroxy-propan-1,1-diphosphonsäure werden mit 6,1 ml Ameisensäure und 4,2 ml einer 38 %igen Formaldehydlösung in Wasser 36 Stunden unter Rückfluss zum Sieden erhitzt. Die Reaktionslösung wird unter vermindertem Druck eingedampft und der Rückstand mit Aceton verdünnt. Man erhält die 3-[N-(3-Phenylpropyl)-N-methyl-amino]-1-hydroxy-propan-1,1-diphosphonsäure in farblosen Kristallen vom Smp. 219° (Zers.).

### Beispiel 4:

In analoger Weise wie in Beispiel 1 beschrieben erhält man ausgehend von 3-[4,4-Di(p-fluorphenyl)butylamino]-propionsäureäthylester über 3-[4,4-Di(p-fluorphenyl)butylamino]-propionsäure-hydrochlorid, Smp. 165-166°, die 3-[4,4-Di(p-fluorphenyl)butylamino]-1-hydroxy-propan-1,1-diphosphonsäure, Smp. 220-222° (Zers.).

Das Ausgangsmaterial kann z.B. folgendermassen hergestellt werden.

26,1 g (0,1 Mol) 4,4-Di(p-fluorphenyl)butylamin und 18,1 g 3-Brompropionsäureäthylester werden mit 21,0 g Kaliumcarbonat in 200 ml 2-Butanon 24 Stunden unter Rühren und Rückfluss zum Sieden erhitzt. Das Reaktionsgemisch wird filtriert und das Filtrat unter vermindertem Druck eingedampft. Man erhält den rohen 3-[4,4-Di(p-fluorphenyl)-butyl-amino]-propionsäureäthylester als Oel.

In analoger Weise erhält man ausgehend von N-(2-Phenyläthyl)-N-methyl-amin und 4-Brombuttersäureäthylester über 4-[N-(2-Phenyläthyl)-N-methyl-amino]-buttersäureäthylester und 4-[N-(2-Phenyläthyl)-N-methyl-amino]-buttersäure-hydrochlorid die 4-[N-(2-Phenyläthyl)-N-methyl-amino]-1-hydroxy-butan-1,1-diphosphonsäure.

### Beispiel 5:

In analoger Weise wie in Beispiel 1 beschrieben erhält man ausgehend von N-[3-(Imidazol-4-yl)propyl]-N-methyl-amin über 3-{N-[3-(Imidazol-4-yl)propyl]-N-methyl-amino}-propionsäureäthylester und 3-{N-[3-(Imidazol-4-yl)propyl]-N-methyl-amino}-propionsäure hydrochlorid die 3-{N-[3-(Imidazol-4-yl)propyl]-N-methyl-amino}-1-hydroxypropan-1,1-diphosphonsäure.

Das Ausgangsmaterial kann z.B. folgendermassen hergestellt werden:
16,8 g (0,12 Mol) 3-[Imidazol-4(5)-yl]propionsäure werden mit 13,1 ml Thionylchlorid 2 Stunden unter Rückfluss zum Sieden erhitzt. Nach dem Abdestillieren des überschüssigen Thionylchlorids verbleibt das 3-[Imidazol-4(5)-yl]propionsaurechlorid-hydrochlorid als halbfeste Masse (Ausbeute 100 %)
23,4 g (0,12 Mol) 3-[Imidazol-4(5)-yl]propionsäurechlorid-hydrochlorid werden in 80 ml Dimethylformamid gelöst und auf -10° gekühlt. Man leitet während 2 Stunden Methylamin-Gas in die Lösung ein, bis die Gewichtszunahme 15 g beträgt. Nach Stehen über Nacht bei Raumtemperatur wird das Reaktionsgemisch unter vermindertem Druck zur Trockne eingedampft. Der Rückstand wird an 220 g Kieselgel mit dem Laufmittel Chloroform/Methanol/konz. Ammoniak (80:20:1) chromatographiert. Die das Produkt enthaltenden Fraktionen liefern aus Tetrahydrofuran farblose Kristalle von 3-[Imidazol-4(5)-yl]propionsäure-(Nmethyl)amid, Smp. 168-170°. Eine Suspension von 2,8 g Lithiumaluminiumhydrid in 200 ml Tetrahydrofuran wird portionenweise unter Rühren mit 10,9 g (0,0718 Mol) 3-[Imidazol-4(5)-yl]propionsäure-(Nmethyl)amid versetzt und anschliessend 30 Stunden unter Rückfluss zum Sieden erhitzt. Dann wird das Reaktionsgemisch auf 0° gekühlt und nacheinander tropfenweise mit 3 ml Wasser, 2,2 ml 10-n Natriumhydroxyd-Lösung und 8,2 ml Wasser versetzt. Der anorganische Niederschlag wird abfiltriert und das Filtrat unter Vakuum eingedampft. Das zurückbleibende Oel ist das rohe N-[Imidazol-4(5)-yl-propyl]-N-methylamin.

### Beispiel 6:

In analoger Weise wie in Beispiel 1 beschrieben kann man ferner ausgehend von bzw. über
3-[4-(4-Methoxyphenyl)butylamino]propionsäure-hydrochlorid, Smp. 150-152°;
3-[3-(4-Methoxyphenyl)propyl-N-methyl-amino]propionsäure-hydrochlorid, Smp. 108-110°;
3-[3-(4-Chlorphenyl)propyl-N-methyl-amino]propionsäure-hydrochlorid, Smp. 128-130°;
3-[3-(3-Methylphenyl)propyl-N-methyl-amino]propionsäure-hydrochlorid, Smp. 97-98°;
3-[3-(Imidazol-4(5)-yl)propyl-N-methyl-amino]propionsäure-dihydrochlorid, halbfeste Masse;
3-[3-(Pyrid-2-yl)propyl-N-methyl-amino]propionsäure-dihydrochlorid, Smp. 157-160° (Zers.);
3-[N-(2-Phenyläthyl-N-methyl-amino]propionsäure-hydrochlorid und Smp. 144-145° (Zers.);
3-[N-(2-Phenyläthyl)amino]propionsäure-hydrochlorid, Smp. 150-152°.
3-[4-(4-Methoxyphenyl)butylamino]-1-hydroxy-propan-1,1-diphosphonsäure, Smp. 160-164°,(Zers.);
3-[3-(4-Methoxyphenyl)propyl-N-methyl-amino]-1-hydroxy-propan-1,1-diphosphonsäure, Smp. 154-156° (Zers.);
3-[3-(4-Chlorphenyl)propyl-N-methyl-amino]-1-hydroxy-propan-1,1-diphosphonsäure, Smp. 162-166° (Zers.);
3-[3-(3-Methylphenyl)propyl-N-methyl-amino]-1-hydroxy-propan-1,1-diphosphonsäure, Smp. 193-195° (Zers.);
3-[3-(Imidazol-4(5)-yl)propyl-N-methyl-amino]-1-hydroxy-propan-1,1-diphosphonsäure, Smp. 130-136° (Zers.);
3-[3-(Pyrid-2-yl)propyl-N-methyl-amino]-1-hydroxy-propan-1,1-di-phosphonsäure, Smp. 142-147° (Zers.);
3-[N-(2-Phenyläthyl)-N-methyl-amino]-1-hydroxy-propan-1,1-di-phosphonsäure, 187-188° (Zers.), und
3-[N-(2-Phenyläthyl)amino]-1-hydroxy-propan-1,1-diphosphonsäure, Smp. 205-206°.

### Beispiel 7:

1,83 g (0,005 Mol) 3-(4-Phenylbutylamino)-1-hydroxy-propan-1,1-diphosphonsäure werden in 10 ml n-Natronlauge gelöst. Die erhaltene Lösung wird unter vermindertem Druck eingeengt und das Produkt durch Zugabe von Methanol kristallisiert. Man erhält das Dinatrium-3-(4-phenylbutylamino)-1-hydroxy-propan-1,1-diphosphonat vom Smp. 313-316° (Zers.).

In analoger Weise erhält man das Dinatrium-3-[N-(3-phenylpropyl)-N-methyl-amino]-1-hydroxy-propan-1,1-diphosphonat vom Smp. 321-325°.

### Beispiel 8:

Tabletten, enthaltend 75 mg Wirkstoff, z.B. 3-[N-(3-Phenylpropyl)-N-methyl-amino]-1-hydroxy-propan-1,1-diphosphonsäure oder ein Salz, z.B. das Dinatriumsalz, davon, können folgendermassen hergestellt werden:

| Bestandteile (für 1000 Tabletten) | |
|---|---|
| Wirkstoff | 75,0 g |
| Lactose | 268,5 g |
| Maisstärke | 22,5 g |
| Polyäthylenglykol 6000 | 5,0 g |
| Talkum | 15,0 g |
| Magnesiumstearat | 4,0 g |
| entmineralisiertes Wasser | q.s. |

### Herstellung:

Die festen Ingredienzien werden zunächst durch ein Sieb mit 0,6 mm Maschenweite getrieben. Dann wird der Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

### Beispiel 9:

Tabletten, enthaltend 10 mg Wirkstoff, z.B. 3-[N-(3-Phenylpropyl)-N-methyl-amino]-1-hydroxy-propan-1,1-diphosphonsäure oder ein Salz, z.B. das Dinatriumsalz davon, können folgendermassen hergestellt werden:

| Zusammensetzung (für 1000 Tabletten) | |
|---|---|
| Wirkstoff | 10,0 g |
| Lactose | 328,5 g |
| Maisstärke | 17,5 g |
| Polyäthylenglykol 6000 | 5,0 g |
| Talkum | 25,0 g |
| Magnesiumstearat | 4,0 g |
| entmineralisiertes Wasser | q.s. |

### Herstellung:

Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

### Beispiel 10:

Gelatinesteckkapseln, enthaltend 100 mg Wirkstoff, z.B. 3-[N-(3-Phenylpropyl)-N-methyl-amino]-1-hydroxy-propan-1,1-diphosphonsäure oder ein Salz, z.B. das Dinatriumsalz davon, können folgendermassen hergestellt werden:

| Zusammensetzung (für 1000 Kapseln) | |
|---|---|
| Wirkstoff | 350,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse 0 (elongated) abgefüllt.

### Beispiel 11:

Eine 0,2 %ige Injektions- bzw. Infusionslösung kann beispielsweise folgendermassen hergestellt werden.

| | |
|---|---|
| Wirkstoff, z.B. 3-[N-(3-Phenylpropyl)-N-methyl-amino]-1-hydroxy-propan-1,1-diphosphonsäure oder eines ihrer Salze, z.B. ihr Dinatriumsalz | 5,0 g |
| Natriumchlorid | 22,5 g |
| Phosphatpuffer pH = 7,4 | 300,0 g |
| Wasser, entmin. | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen (enthaltend je 2,0 bzw. 5,0 mg Wirkstoff abgefüllt.

## Patentansprüche (Patentansprüche für folgende(n) Vertragsstaat(en): AT, BE, CH, DE, FR, GB, IT, LI, LU, NL, SE)

1. Aromatisch substituierte Alkylaminoalkandiphosphonsäuren der Formel 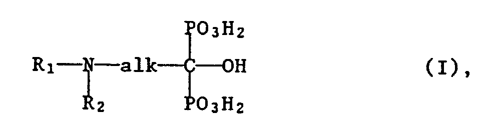 worin R₁ einen durch einen unsubstituierten oder durch Niederalkyl, Niederalkoxy, Niederalkylthio und/oder Halogen substituierten 6-gliedrigen monocyclischen oder aus 5- oder 6-gliedrigen Ringen aufgebauten bicyclischen Aryl-bzw. als Heteroatom(e) 1 oder 2 N-Atome, 1 O-oder S-Atom, 1 N- und 1 O-Atom oder 1 N- und 1 S-Atom aufweisenden Heteroarylrest mono- oder disubstituierten Niederalkyl- oder Niederalkenylrest bedeutet, R₂ Wasserstoff, Niederalkyl oder Niederalkenyl darstellt und alk für Niederalkylen steht, mit der Massgabe, dass R₂ Wasserstoff oder C₁-C₃-Alkyl ist und R₁ im aliphatischen Teil 2 oder 3 C-Atome aufweist, wenn R₁ durch unsubstituiertes Phenyl monosubstituiert ist, und ihre Salze; wobei mit "Nieder" bezeichnete Reste höchstens 7 C-Atome aufweisen.

2. Verbindungen gemäss Anspruch 1 der Formel I, worin R₁ einen durch einen unsubstituierten oder durch Niederalkyl, Niederalkoxy, Niederalkylthio und/oder Halogen substituierten 6-gliedrigen monocyclischen oder aus 5- und/oder 6-gliedrigen Ringen aufgebauten bicyclischen Arylrest mono- oder disubstituierten oder durch einen 5-oder 6-gliedrigen, als Heteroatome 1 O- oder S-Atom oder 1 oder 2 N-Atome aufweisenden monocyclischen Heteroarylrest monosubstituierten Niederalkylrest bedeutet, R₂ Wasserstoff oder einen Niederalkylrest darstellt und alk für Niederalkylen steht, mit der Massgabe, dass R₂ Wasserstoff ist, wenn R₁ einen unsubstituierten Benzyl- oder Phenyl-C₃-C₇-alkylrest bedeutet, bzw. R₂ Wasserstoff oder Niederalkyl mit 1 oder 2 C-Atomen ist, wenn R₁ einen Phenyläthylrest bedeutet, und ihre Salze.

3. Verbindungen gemäss Anspruch 1 der Formel I, worin R₁ einen Rest der Formel 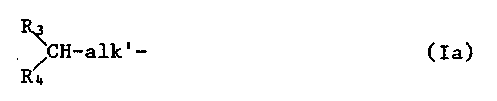 bedeutet, in der R₃ einen aromatischen Rest R_{A}, R₄ Wasserstoff oder einen aromatischen Rest R_{B} und alk' Niederalkylen bedeutet, wobei R_{A} bzw.R_{B} jeweils einen unsubstituierten oder durch Niederalkyl, Niederalkoxy, Niederalkylthio und/oder Halogen substituierten 5- oder 6-gliedrigen monocyclischen oder aus 5- oder 6-gliedrigen Ringen aufgebauten bicyclischen Aryl- bzw. als Heteroatom(e) 1 oder 2 N-Atome, 1 O- oder S-Atom, 1 N- und 1 O-Atom oder 1 N- und 1 S-Atom aufweisenden Heteroarylrest darstellt, R₂ Wasserstoff, Niederalkyl oder Niederalkenyl bedeutet, und alk für Niederalkylen steht, und ihre Salze.

4. Verbindungen gemäss Anspruch 1 der Formel I, worin R₁ einen Rest der Formel 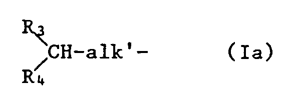 bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest darstellt, alk' für C₁-C₅-Alkylen steht, R₂ für Wasserstoff steht und alk geradkettiges C₂-C₄-Alkylen darstellt, und ihre Salze.

5. Verbindungen gemäss Anspruch 1 der Formel I, worin R₁ einen Rest der Formel 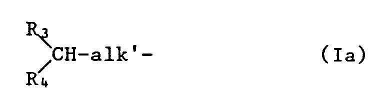 bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₅-Alkoxy, C₁-C₄-Alkylthio und/oder Halogen der Atomnummer bis und mit 35 mono- oder disubstituierten Phenylrest darstellt, alk' für geradkettiges C₁-C₅-Alkylen steht, R₂ C₁-C₄-Alkyl bzw. C₂-C₄-Alkenyl darstellt und alk geradkettiges C₂-C₄-Alkylen darstellt, mit der Massgabe, dass alk' Methylen und R₂ C₁-C₂-Alkyl ist, wenn R₃ unsubstituiertes Phenyl und R₄ Wasserstoff bedeutet, und ihre Salze.

6. Verbindungen gemäss Anspruch 1 der Formel I, worin R₁ einen Rest der Formel 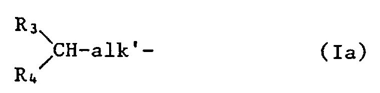 darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ für Wasserstoff steht, alk' geradkettiges, terminal gebundenes C₂-C₄-Alkylen bedeutet, R₂ Wasserstoff bedeutet oder, sofern alk' C₁-C₂-Alkylen bedeutet, für C₁-C₃-Alkyl steht und alk C₂-C₃-Alkylen darstellt, und ihre Salze.

7. Verbindungen gemäss Anspruch 1 der Formel I, worin R₁ unsubstituiertes oder im Phenylteil durch C₁-C₄-Alkyl, C₁-C₄-Alkoxy und/oder Halogen der Atomnummer bis und mit 35 mono- oder disubstituiertes Mono- oder Diphenyl-C₂-C₆-alkyl oder Imidazolyl-, Thienyl- oder Pyridyl-C₂-C₆-alkyl bedeutet, R₂ für Wasserstoff oder C₁-C₄-Alkyl steht und alk C₂-C₃-Alkylen darstellt, mit der Massgabe, dass R₂ Wasserstoff ist, wenn R₁ unsubstituiertes Phenyl-C₃-C₄-alkyl bedeutet, bzw. R₂ Wasserstoff oder C₁-C₂-Alkyl ist, wenn R₁ einen unsubstituierten Phenyläthylrest darstellt, und ihre Salze.

8. Verbindungen gemäss Anspruch 1 der Formel I, worin R₁ einen Rest der Formel 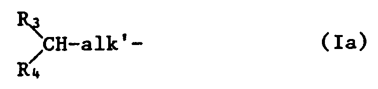 darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ Wasserstoff ist, alk' geradkettiges, terminal gebundenes C₂-C₄-Alkylen bedeutet, R₂ Wasserstoff bedeutet und alk C₂-C₃-Alkylen darstellt, und ihre Salze.

9. 3-[N-(3-Phenylpropyl)-N-methyl-amino]-1-hydroxy-propan-1,1-diphosphonsäure gemäss Anspruch 1 oder ein Salz davon.

10. 3-(3-Phenylpropylamino)-1-hydroxy-propan-1,1-diphosphonsäure gemäss Anspruch 1 oder ein Salz davon.

11. 3-[N-(3-Phenylpropyl)-N-äthyl-amino]-1-hydroxy-propan-1,1-diphosphonsäure gemäss Anspruch 1 oder ein Salz davon.

12. 3-[3-(Pyrid-3-yl)-propylamino]-1-hydroxy-propan-1,1-diphosphonsäure gemäss Anspruch 1 oder ein Salz davon.

13. 3-(4-Phenylbutylamino)-1-hydroxy-propan-1,1-diphosphonsäure gemäss Anspruch 1 oder ein Salz davon.

14. 3-[4-(4-Methoxyphenyl)butylamino]-1-hydroxy-propan-1,1-diphosphonsäure gemäss Anspruch 1 oder ein Salz davon.

15. 3-[3-(4-Methoxyphenyl)propyl-N-methyl-amino]-1-hydroxy-propan-1,1-diphosphonsäure gemäss Anspruch 1 oder ein Salz davon.

16. 3-[3-(4-Chlorphenyl)propyl-N-methyl-amino]-1-hydroxy-propan-1,1-diphosphonsäure gemäss Anspruch 1 oder ein Salz davon.

17. 3-[3-(3-Methylphenyl)propyl-N-methyl-amino]-1-hydroxy-propan-1,1-diphosphonsäure gemäss Anspruch 1 oder ein Salz davon.

18. 3-[3-(Pyrid-2-yl)propyl-N-methyl-amino]-1-hydroxy-propan-1,1-diphosphonsäure gemäss Anspruch 1 oder ein Salz davon.

19. Eine Verbindung gemäss einem der Ansprüche 1, 3, 6 und 8-12 in freier Form oder in pharmazeutisch verwendbarer Salzform zur Anwendung in einem Verfahren zur therapeutischen Behandlung des menschlichen oder tierischen Körpers.

20. Eine Verbindung gemäss einem der Ansprüche 2, 4, 5, 7 und 13-18 in freier Form oder in pharmazeutisch verwendbarer Salzform zur Anwendung in einem Verfahren zur therapeutischen Behandlung des menschlichen oder tierischen Körpers.

21. Pharmazeutische Präparate enthaltend eine Verbindung gemäss einem der Ansprüche 1, 3, 6, 8-12 und 19 neben üblichen pharmazeutischen Trägerstoffen.

22. Pharmazeutische Präparate enthaltend eine Verbindung gemäss einem der Ansprüche 2, 4, 5, 7, 13-18 und 20 neben üblichen pharmazeutischen Trägerstoffen.

23. Verfahren zur Herstellung aromatisch substituierter Alkylaminoalkandiphosphonsäuren gemäss Anspruch 1, oder Salzen davon dadurch gekennzeichnet, dass man
a) in einer Verbindung der Formel 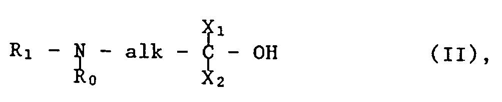 worin R₀ einen Rest R₂ oder eine Aminoschutzgruppe darstellt und X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln 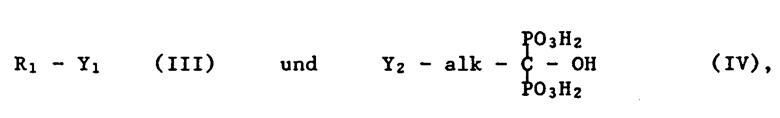 worin einer der Reste Y₁ und Y₂ eine reaktionsfähige veresterte Hydroxygruppe und der andere eine Gruppe der Formel -N(R₀)-H bedeutet, in der R₀ einen Rest R₂ oder eine Aminoschutzgruppe darstellt, oder Salze davon, miteinander umsetzt, oder
c) eine Verbindung der Formel 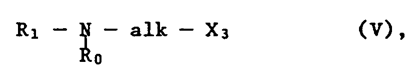 worin R₀ einen Rest R₂ oder eine Aminoschutzgruppe darstellt und X₃ Carboxy, Carbamyl oder Cyano, insbesondere Carboxy oder Cyano, bedeutet, mit phosphoriger Säure und Phosphortrichlorid umsetzt, das Primärprodukt hydrolysiert und in einem ausgehend von Verbindungen der Formel V, worin X₃ Cyano oder Carbamyl ist, erhaltenen Zwischenprodukt der Formel 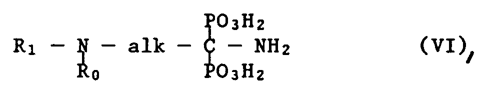 bzw. einem Salz davon, die Aminogruppe durch Behandlung mit salpetriger Säure durch Hydroxy ersetzt; jeweils die Aminoschutzgruppe, sofern vorhanden, abspaltet und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

24. Verwendung einer Verbindung gemäss einem der Ansprüche 1 bis 20 zur Herstellung eines zur Behandlung von Störungen des Calciumstoffwechsels und damit verbundenen Erkrankungen geeigneten Arzneimittels.

## Patentansprüche (Patentansprüche für folgende(n) Vertragsstaat(en): ES, GR)

1. Verfahren zur Herstellung aromatisch substituierter Alkylaminoalkandiphosphonsäuren der Formel 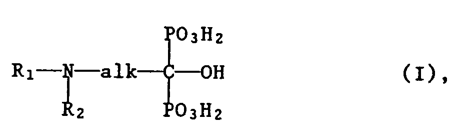 worin R₁ einen durch einen unsubstituierten oder durch Niederalkyl, Niederalkoxy, Niederalkylthio und/oder Halogen substituierten 6-gliedrigen monocyclischen oder aus 5- oder 6-gliedrigen Ringen aufgebauten bicyclischen Aryl- bzw. als Heteroatom(e) 1 oder 2 N-Atome, 1 O-oder S-Atom, 1 N- und 1 O-Atom oder 1 N- und 1 S-Atom aufweisenden Heteroarylrest mono- oder disubstituierten Niederalkyl- oder Niederalkenylrest bedeutet, R₂ Wasserstoff, Niederalkyl oder Niederalkenyl darstellt und alk für Niederalkylen steht, mit der Massgabe, dass R₂ Wasserstoff oder C₁-C₃-Alkyl ist und R₁ im aliphatischen Teil 2 oder 3 C-Atome aufweist, wenn R₁ durch unsubstituiertes Phenyl monosubstituiert ist, wobei mit "Nieder" bezeichnete Reste höchstens 7 C-Atome aufweisen, oder Salzen davon, dadurch gekennzeichnet, dass man
a) in einer Verbindung der Formel 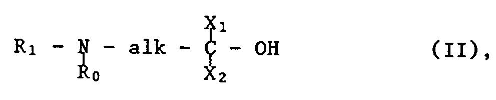 worin R₀ einen Rest R₂ oder eine Aminoschutzgruppe darstellt und X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln 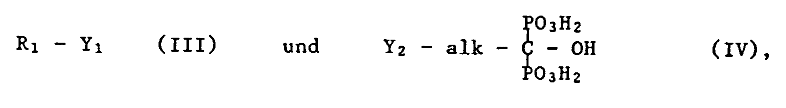 worin einer der Reste Y₁ und Y₂ eine reaktionsfähige veresterte Hydroxygruppe und der andere eine Gruppe der Formel -N(R₀)-H bedeutet, in der R₀ einen Rest R₂ oder eine Aminoschutzgruppe darstellt, oder Salze davon, miteinander umsetzt, oder
c) eine Verbindung der Formel 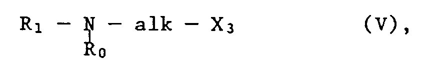 worin R₀ einen Rest R₂ oder eine Aminoschutzgruppe darstellt und X₃ Carboxy, Carbamyl oder Cyano, insbesondere Carboxy oder Cyano, bedeutet, mit phosphoriger Säure und Phosphortrichlorid umsetzt, das Primärprodukt hydrolysiert und in einem ausgehend von Verbindungen der Formel V, worin X₃ Cyano oder Carbamyl ist, erhaltenen Zwischenprodukt der Formel 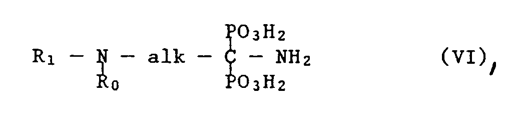 bzw. einem Salz davon, die Aminogruppe durch Behandlung mit salpetriger Säure durch Hydroxy ersetzt; jeweils die Aminoschutzgruppe, sofern vorhanden, abspaltet und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

2. Verfahren gemäss Anspruch 1 zur Herstellung von Verbindungen der Formel I, worin R₁ einen durch einen unsubstituierten oder durch Niederalkyl, Niederalkoxy, Niederalkylthio und/oder Halogen substituierten 6-gliedrigen monocyclischen oder aus 5- und/oder 6-gliedrigen Ringen aufgebauten bicyclischen Arylrest mono- oder disubstituierten oder durch einen 5- oder 6-gliedrigen, als Heteroatome 1 O- oder S-Atom oder 1 oder 2 N-Atome aufweisenden monocyclischen Heteroarylrest monosubstituierten Niederalkylrest bedeutet, R₂ Wasserstoff oder einen Niederalkylrest darstellt und alk für Niederalkylen steht, mit der Massgabe, dass R₂ Wasserstoff ist, wenn R₁ einen unsubstituierten Benzyl- oder Phenyl-C₃-C₇-alkylrest bedeutet, bzw. R₂ Wasserstoff oder Niederalkyl mit 1 oder 2 C-Atomen ist, wenn R₁ einen Phenyläthylrest bedeutet, und ihrer Salze.

3. Verfahren gemäss Anspruch 1 zur Herstellung von Verbindungen der Formel I, worin R₁ einen Rest der Formeln 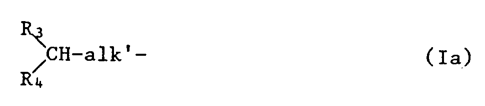 bedeutet, in der R₃ einen aromatischen Rest R_{A}, R₄ Wasserstoff oder einen aromatischen Rest R_{B} und alk' Niederalkylen bedeutet, wobei R_{A} bzw.R_{B} jeweils einen unsubstituierten oder durch Niederalkyl, Niederalkoxy, Niederalkylthio und/oder Halogen substituierten 5- oder 6-gliedrigen monocyclischen oder aus 5- oder 6-gliedrigen Ringen aufgebauten bicyclischen Aryl- bzw. als Heteroatom(e) 1 oder 2 N-Atome, 1 O- oder S-Atom, 1 N- und 1 O-Atom oder 1 N- und 1 S-Atom aufweisenden Heteroarylrest darstellt, R₂ Wasserstoff, Niederalkyl oder Niederalkenyl bedeutet, und alk für Niederalkylen steht, und ihrer Salze.

4. Verfahren gemäss Anspruch 1 zur Herstellung von Verbindungen der Formel I, worin R₁ einen Rest der Formel 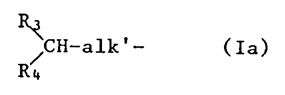 bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest darstellt, alk' für C₁-C₅-Alkylen steht, R₂ für Wasserstoff steht und alk geradkettiges C₂-C₄-Alkylen darstellt, und ihrer Salze.

5. Verfahren gemäss Anspruch 1 zur Herstellung von Verbindungen der Formel I, worin R₁ einen Rest der Formel 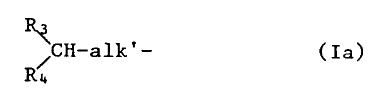 bedeutet, in der R₃ einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenyl-, Thienyl-, Pyridyl-, Imidazolyl- oder Naphthylrest bedeutet, R₄ Wasserstoff oder, sofern R₃ für einen unsubstituierten oder durch C₁-C₄-Alkyl, C₁-C₄-Alkoxy, C₁-C₄-Alkylthio und/oder Halogen der Atomnummer bis und mit 35 mono- oder disubstituierten Phenylrest steht, ebenfalls einen unsubstituierten oder durch C₁-C₄-Alkyl, C₁-C₅-Alkoxy, C₁-C₄-Alkylthio und/oder Halogen der Atomnummer bis und mit 35 mono- oder disubstituierten Phenylrest darstellt, alk' für geradkettiges C₁-C₅-Alkylen steht, R₂ C₁-C₄-Alkyl bzw. C₂-C₄-Alkenyl darstellt und alk geradkettiges C₂-C₄-Alkylen darstellt, mit der Massgabe, dass alk' Methylen und R₂ C₁-C₂-Alkyl ist, wenn R₃ unsubstituiertes Phenyl und R₄ Wasserstoff bedeutet, und ihrer Salze.

6. Verfahren gemäss Anspruch 1 zur Herstellung von Verbindungen der Formel I, worin R₁ einen Rest der Formel 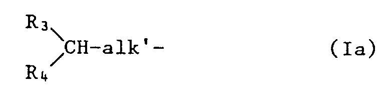 darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ für Wasserstoff steht, alk' geradkettiges, terminal gebundenes C₂-C₄-Alkylen bedeutet, R₂ Wasserstoff bedeutet oder, sofern alk' C₁-C₂-Alkylen bedeutet, für C₁-C₃-Alkyl steht und alk C₂-C₃-Alkylen darstellt, und ihrer Salze.

7. Verfahren gemäss Anspruch 1 zur Herstellung von Verbindungen der Formel I, worin R₁ unsubstituiertes oder im Phenylteil durch C₁-C₄-Alkyl, C₁-C₄-Alkoxy und/oder Halogen der Atomnummer bis und mit 35 mono- oder disubstituiertes Mono-oder Diphenyl-C₂-C₆-alkyl oder Imidazolyl-, Thienyl- oder Pyridyl-C₂-C₆-alkyl bedeutet, R₂ für Wasserstoff oder C₁-C₄-Alkyl steht und alk C₂-C₃-Alkylen darstellt, mit der Massgabe, dass R₂ Wasserstoff ist, wenn R₁ unsubstituiertes Phenyl-C₃-C₄-alkyl bedeutet, bzw. R₂ Wasserstoff oder C₁-C₂-Alkyl ist, wenn R₁ einen unsubstituierten Phenyläthylrest darstellt, und ihrer Salze.

8. Verfahren gemäss Anspruch 1 zur Herstellung von Verbindungen der Formel I, worin R₁ einen Rest der Formel 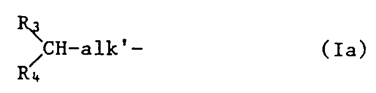 darstellt, in der R₃ unsubstituiertes oder durch C₁-C₄-Alkyl, C₁-C₄-Alkoxy und/oder Halogen der Atomnummer bis und mit 35 mono- oder disubstituiertes Phenyl bedeutet, R₄ Wasserstoff ist, alk' geradkettiges, terminal gebundenes C₂-C₄-Alkylen bedeutet, R₂ Wasserstoff bedeutet und alk C₂-C₃-Alkylen darstellt, und ihrer Salze.

9. Verfahren zur Herstellung von 3-[N-(3-Phenylpropyl)-N-methyl-amino]-1-hydroxy-propan-1,1-diphosphonsäure oder eines Salzes davon.

10. Verfahren zur Herstellung von 3-(3-Phenylpropylamino)-1-hydroxy-propan-1,1-diphosphonsäure oder eines Salzes davon.

11. Verfahren zur Herstellung von 3-[N-(3-Phenylpropyl)-N-äthyl-amino]-1-hydroxy-propan-1,1-diphosphonsaure oder eines Salzes davon.

12. Verfahren zur Herstellung von 3-[3-(Pyrid-3-yl)-propylamino]-1-hydroxy-propan-1,1-diphosphonsäure oder eines Salzes davon.

13. Verfahren zur Herstellung von 3-(4-Phenylbutylamino)-1-hydroxy-propan-1,1-diphosphonsäure oder eines Salzes davon.

14. Verfahren zur Herstellung von 3-[4-(4-Methoxyphenyl)butylamino]-1-hydroxy-propan-1,1-diphosphonsäure oder eines Salzes davon.

15. Verfahren Zur Herstellung von 3-[3-(4-Methoxyphenyl)propyl-N-methyl-amino]-1-hydroxy-propan-1,1- diphosphonsäure oder eines Salzes davon.

16. Verfahren zur Herstellung von 3-[3-(4-Chlorphenyl)propyl-N-methyl-amino]-1-hydroxy-propan-1,1-diphosphonsäure oder eines Salzes davon.

17. Verfahren zur Herstellung von 3-[3-(3-Methylphenyl)propyl-N-methyl-amino]-1-hydroxy-propan-1,1-di-phosphonsäure oder eines Salzes davon.

18. Verfahren zur Herstellung von 3-[3-(Pyrid-2-yl)propyl-N-methyl-amino]-1-hydroxy-propan-1,1-di-phosphonsäure oder eines Salzes davon.

19. Verfahren zur Herstellung von pharmazeutischen Präparaten, dadurch gekennzeichnet, dass man eine Verbindung, erhältlich gemäss einem der Ansprüche 1, 3, 6 und 8-12, mit üblichen pharmazeutischen Trägerstoffen vermischt.

20. Verfahren zur Herstellung von pharmazeutischen Präparaten, dadurch gekennzeichnet, dass man eine Verbindung, erhältlich gemäss einem der Ansprüche 2, 4, 5, 7, und 13-18, mit üblichen pharmazeutischen Trägerstoffen vermischt.

## Claims (Claims for the following Contracting State(s): AT, BE, CH, DE, FR, GB, IT, LI, LU, NL, SE)

1. An aromatically substituted alkylaminoalkanediphosphonic acid of formula 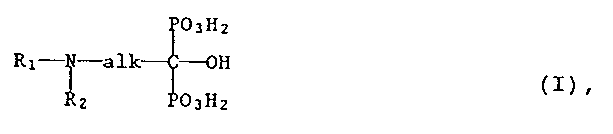 wherein R₁ is a lower alkyl or lower alkenyl radical that is mono- or di-substituted by a 6-membered monocyclic aryl radical or by a bicyclic aryl radical composed of 5- or 6-membered rings or by a 6-membered monocyclic heteroaryl radical or by a bicyclic heteroaryl radical composed of 5- or 6-membered rings, which heteroaryl radicals contain as hetero atom(s) 1 or 2 N-atom(s), 1 O-atom or S-atom, 1 N-atom and 1 O-atom, or 1 N-atom and 1 S-atom, and which aryl and heteroaryl radicals are unsubstituted or substituted by lower alkyl, lower alkoxy, lower alkylthio and/or by halogen, R₂ is hydrogen, lower alkyl or lower alkenyl, and alk is lower alkylene, with the proviso that R₂ is hydrogen or C₁-C₃alkyl and R₁ has 2 or 3 carbon atoms in the aliphatic moiety when R₁ is mono-substituted by unsubstituted phenyl, or a salt thereof, radicals designated "lower" containing a maximum of 7 carbon atoms.

2. A compound according to claim 1 of formula I wherein R₁ is a lower alkyl radical that is mono- or di-substituted by a 6-membered monocyclic aryl radical or by a bicyclic aryl radical composed of 5- and/or 6-membered rings, which aryl radicals are unsubstituted or substituted by lower alkyl, lower alkoxy, lower alkylthio and/or by halogen, or is a lower alkyl radical that is mono-substituted by a 5- or 6-membered monocyclic heteroaryl radical that contains as hetero atom(s) 1 O-atom or S-atom or 1 or 2 N-atom(s), R₂ is hydrogen or a lower alkyl radical and alk is lower alkylene, with the proviso that R₂ is hydrogen when R₁ is an unsubstituted benzyl- or phenyl-C₃-C₇alkyl radical, or that R₂ is hydrogen or lower alkyl containing 1 or 2 carbon atom(s) when R₁ is a phenethyl radical, or a salt thereof.

3. A compound according to claim 1 of formula I wherein R₁ is a radical of formula 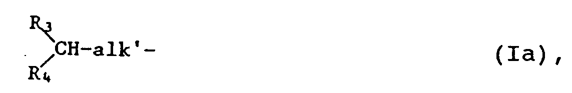 wherein R₃ is an aromatic radical R_{A}, R₄ is hydrogen or an aromatic radical R_{B} and alk' is lower alkylene, R_{A} and R_{B} each being a 5- or 6-membered monocyclic aryl radical or a bicyclic aryl radical composed of 5- or 6-membered rings or a 5- or 6-membered monocyclic heteroaryl radical or a bicyclic heteroaryl radical composed of 5- or 6-membered rings, which heteroaryl radicals contain as hetero atom(s) 1 or 2 N-atom(s), 1 O-atom or S-atom, 1 N-atom and 1 O-atom or 1 N-atom and 1 S-atom, and which aryl and heteroaryl radicals are unsubstituted or substituted by lower alkyl, lower alkoxy, lower alkylthio and/or by halogen, R₂ is hydrogen, lower alkyl or lower alkenyl, and alk is lower alkylene, or a salt thereof.

4. A compound according to claim 1 of formula I wherein R₁ is a radical of formula 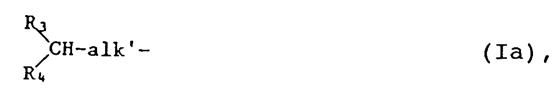 wherein R₃ is a phenyl, thienyl, pyridyl, imidazolyl or naphthyl radical that is unsubstituted or mono- or di-substituted by C₁-C₄alkyl, C₁-C₄alkoxy, C₁-C₄alkylthio and/or by halogen having an atomic number of up to and including 35, R₄ is hydrogen or, when R₃ is a phenyl radical that is unsubstituted or mono- or di-substituted by C₁-C₄alkyl, C₁-C₄alkoxy, C₁-C₄alkylthio and/or by halogen having an atomic number of up to and including 35, R₄ is likewise a phenyl radical that is unsubstituted or mono- or di-substituted by C₁-C₄alkyl, C₁-C₄alkoxy, C₁-C₄alkylthio and/or by halogen having an atomic number of up to and including 35, alk' is C₁-C₅alkylene, R₂ is hydrogen, and alk is straight-chained C₂-C₄alkylene, or a salt thereof.

5. A compound according to claim 1 of formula I wherein R₁ is a radical of formula 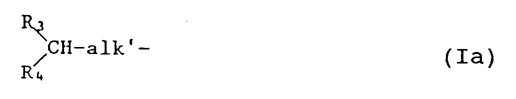 wherein R₃ is a phenyl, thienyl, pyridyl, imidazolyl or naphthyl radical that is unsubstituted or mono- or di-substituted by C₁-C₄alkyl, C₁-C₄alkoxy, C₁-C₄alkylthio and/or by halogen having an atomic number of up to and including 35, R₄ is hydrogen or, when R₃ is a phenyl radical that is unsubstituted or mono- or di-substituted by C₁-C₄alkyl, C₁-C₄alkoxy, C₁-C₄alkylthio and/or by halogen having an atomic number of up to and including 35, R₄ is likewise a phenyl radical that is unsubstituted or mono- or di-substituted by C₁-C₄alkyl, C₁-C₅alkoxy, C₁-C₄alkylthio and/or by halogen having an atomic number of up to and including 35, alk' is straight-chained C₁-C₅alkylene, R₂ is C₁-C₄alkyl or C₂-C₄alkenyl, and alk is straight-chained C₂-C₄alkylene, with the proviso that alk' is methylene and R₂ is C₁-C₂alkyl when R₃ is unsubstituted phenyl and R₄ is hydrogen, or a salt thereof.

6. A compound according to claim 1 of formula I wherein R₁ is a radical of formula 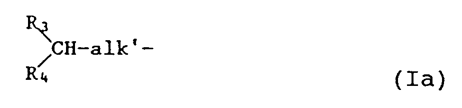 wherein R₃ is phenyl that is unsubstituted or mono- or di-substituted by C₁-C₄alkyl, C₁-C₄alkoxy and/or by halogen having an atomic number of up to and including 35, R₄ is hydrogen, alk' is straight-chained, terminally bonded C₂-C₄alkylene, R₂ is hydrogen or, when alk' is C₁-C₂alkylene, is C₁-C₃alkyl, and alk is C₂-C₃alkylene, or a salt thereof.

7. A compound according to claim 1 of formula I wherein R₁ is mono- or di-phenyl-C₂-C₆alkyl that is unsubstituted or mono- or di-substituted in the phenyl moiety by C₁-C₄alkyl, C₁-C₄alkoxy and/or by halogen having an atomic number of up to and including 35, or is imidazolyl-, thienyl- or pyridyl-C₂-C₆alkyl, R₂ is hydrogen or C₁-C₄alkyl, and alk is C₂-C₃alkylene, with the proviso that R₂ is hydrogen when R₁ is unsubstituted phenyl-C₃-C₄alkyl, or R₂ is hydrogen or C₁-C₂alkyl when R₁ is an unsubstituted phenethyl radical, or a salt thereof.

8. A compound according to claim 1 of formula I wherein R₁ is a radical of formula 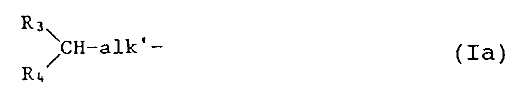 wherein R₃ is phenyl that is unsubstituted or mono- or di-substituted by C₁-C₄alkyl, C₁-C₄alkoxy and/or by halogen having an atomic number of up to and including 35, R₄ is hydrogen, alk' is straight-chained, terminally bonded C₂-C₄alkylene, R₂ is hydrogen and alk is C₂-C₃alkylene, or a salt thereof.

9. 3-[N-(3-phenylpropyl)-N-methyl-amino]-1-hydroxy-propane-1,1-diphosphonic acid according to claim 1 or a salt thereof.

10. 3-(3-Phenylpropylamino)-1-hydroxy-propane-1,1-diphosphonic acid according to claim 1 or a salt thereof.

11. 3-[N-(3-phenylpropyl)-N-ethyl-amino]-1-hydroxy-propane-1,1-diphosphonic acid according to claim 1 or a salt thereof.

12. 3-[3-(Pyrid-3-yl)-propylamino]-1-hydroxy-propane-1,1-diphosphonic acid according to claim 1 or a salt thereof.

13. 3-(4-Phenylbutylamino)-1-hydroxy-propane-1,1-diphosphonic acid according to claim 1 or a salt thereof.

14. 3-[4-(4-Methoxyphenyl)butylamino]-1-hydroxy-propane-1,1-diphosphonic acid according to claim 1 or a salt thereof.

15. 3-[3-(4-Methoxyphenyl)propyl-N-methyl-amino]-1-hydroxy-propane-1,1-diphosphonic acid according to claim 1 or a salt thereof.

16. 3-[3-(4-Chlorophenyl)propyl-N-methyl-amino]-1-hydroxy-propane-1,1-diphosphonic acid according to claim 1 or a salt thereof.

17. 3-[3-(3-Methylphenyl)propyl-N-methyl-amino]-1-hydroxy-propane-1,1-diphosphonic acid according to claim 1 or a salt thereof.

18. 3-[3-(Pyrid-2-yl)propyl-N-methyl-amino]-1-hydroxy-propane-1,1-diphosphonic acid according to claim 1 or a salt thereof.

19. A compound according to any one of claims 1, 3, 6 and 8 to 12 in free form or in the form of a pharmaceutically acceptable salt for use in a method for the therapeutic treatment of the human or animal body.

20. A compound according to any one of claims 2, 4, 5, 7 and 13 to 18 in free form or in the form of a pharmaceutically acceptable salt for use in a method for the therapeutic treatment of the human or animal body.

21. A pharmaceutical composition comprising a compound according to any one of claims 1, 3, 6, 8 to 12 and 19, together with customary pharmaceutical carrier(s).

22. A pharmaceutical composition comprising a compound according to any one of claims 2, 4, 5, 7, 13 to 18 and 20, together with customary pharmaceutical carrier(s).

23. A process for the preparation of an aromatically substituted alkylaminoalkanediphosphonic acid according to claim 1, or a salt thereof, which comprises
a) in a compound of formula 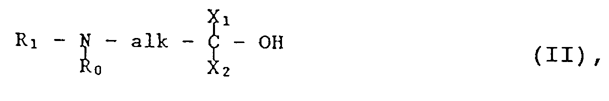 wherein R₀ is a radical R₂ or an amino-protecting group and X₁ is a functionally modified phosphono group and X₂ is a free or functionally modified phosphono group, converting functionally modified phosphono X₁ and, where appropriate, X₂ into a free phosphono group, or
b) reacting compounds of formulae 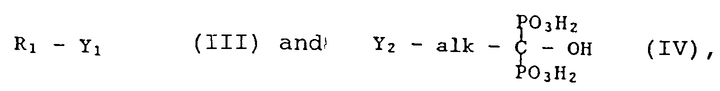 wherein one of the radicals Y₁ and Y₂ is a reactive esterified hydroxy group and the other is a group of formula -N(R₀)-H wherein R₀ is a radical R₂ or an amino-protecting group, or salts thereof, with each other, or
c) reacting a compound of formula 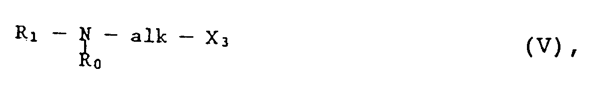 wherein R₀ is a radical R₂ or an amino-protecting group and X₃ is carboxy, carbamoyl or cyano, especially carboxy or cyano, with phosphorous acid and phosphorus trichloride, hydrolysing the primary product and, in an intermediate of formula 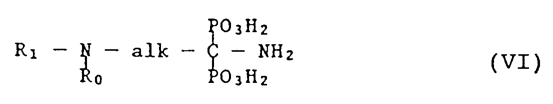 obtained starting from compounds of formula V wherein X₃ is cyano or carbamoyl, or in a salt thereof, replacing the amino group by hydroxy by treatment with nitrous acid; and in each case removing the amino-protecting group if present and, if desired, converting a resulting compound into a different compound of formula I and/or converting a resulting free compound into a salt or converting a resulting salt into the free compound or into a different salt.

24. The use of a compound according to any one of claims 1 to 20 for the preparation of a medicament suitable for the treatment of calcium metabolism disorders and of diseases that are associated therewith.

## Claims (Claims for the following Contracting State(s): ES, GR)

1. A process for the preparation of an aromatically substituted alkylaminoalkanediphosphonic acid of formula 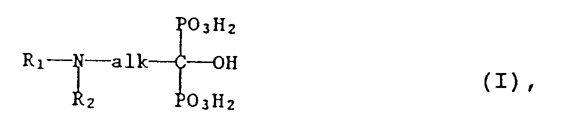 wherein R₁ is a lower alkyl or lower alkenyl radical that is mono- or di-substituted by a 6-membered monocyclic aryl radical or by a bicyclic aryl radical composed of 5- or 6-membered rings or by a 6-membered monocyclic heteroaryl radical or by a bicyclic heteroaryl radical composed of 5- or 6-membered rings, which heteroaryl radicals contain as hetero atom(s) 1 or 2 N-atom(s), 1 O-atom or S-atom, 1 N-atom and 1 O-atom, or 1 N-atom and 1 S-atom, and which aryl and heteroaryl radicals are unsubstituted or substituted by lower alkyl, lower alkoxy, lower alkylthio and/or by halogen, R₂ is hydrogen, lower alkyl or lower alkenyl, and alk is lower alkylene, with the proviso that R₂ is hydrogen or C₁-C₃alkyl and R₁ has 2 or 3 carbon atoms in the aliphatic moiety when R₁ is mono-substituted by unsubstituted phenyl, radicals designated "lower" containing a maximum of 7 carbon atoms, or a salt thereof, which comprises
a) in a compound of formula 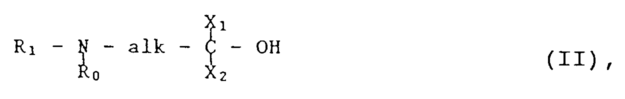 wherein R₀ is a radical R₂ or an amino-protecting group and X₁ is a functionally modified phosphono group and X₂ is a free or functionally modified phosphono group, converting functionally modified phosphono X₁ and, where appropriate, X₂ into a free phosphono group, or
b) reacting compounds of formulae 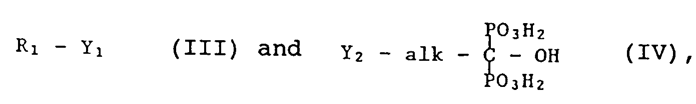 wherein one of the radicals Y₁ and Y₂ is a reactive esterified hydroxy group and the other is a group of formula -N(R₀)-H wherein R₀ is a radical R₂ or an amino-protecting group, or salts thereof, with each other, or
c) reacting a compound of formula 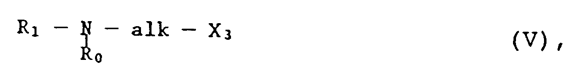 wherein R₀ is a radical R₂ or an amino-protecting group and X₃ is carboxy, carbamoyl or cyano, especially carboxy or cyano, with phosphorous acid and phosphorus trichloride, hydrolysing the primary product and, in an intermediate of formula 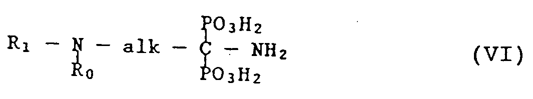 obtained starting from compounds of formula V wherein X₃ is cyano or carbamoyl, or in a salt thereof, replacing the amino group by hydroxy by treatment with nitrous acid; and in each case removing the amino-protecting group if present and, if desired, converting a resulting compound into a different compound of formula I and/or converting a resulting free compound into a salt or converting a resulting salt into the free compound or into a different salt.

2. A process according to claim 1 for the preparation of a compound of formula I wherein R₁ is a lower alkyl radical that is mono- or di-substituted by a 6-membered monocyclic aryl radical or by a bicyclic aryl radical composed of 5- and/or 6-membered rings, which aryl radicals are unsubstituted or substituted by lower alkyl, lower alkoxy, lower alkylthio and/or by halogen, or is a lower alkyl radical that is mono-substituted by a 5- or 6-membered monocyclic heteroaryl radical that contains as hetero atom(s) 1 O-atom or S-atom or 1 or 2 N-atom(s), R₂ is hydrogen or a lower alkyl radical and alk is lower alkylene, with the proviso that R₂ is hydrogen when R₁ is an unsubstituted benzyl- or phenyl-C₃-C₇alkyl radical, or that R₂ is hydrogen or lower alkyl containing 1 or 2 carbon atom(s) when R₁ is a phenethyl radical, or a salt thereof.

3. A process according to claim 1 for the preparation of a compound of formula I wherein R₁ is a radical of formula 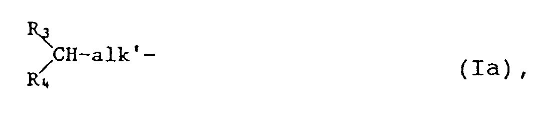 wherein R₃ is an aromatic radical R_{A}, R₄ is hydrogen or an aromatic radical R_{B} and alk' is lower alkylene, R_{A} and R_{B} each being a 5- or 6-membered monocyclic aryl radical or a bicyclic aryl radical composed of 5- or 6-membered rings or a 5- or 6-membered monocyclic heteroaryl radical or a bicyclic heteroaryl radical composed of 5- or 6-membered rings, which heteroaryl radicals contain as hetero atom(s) 1 or 2 N-atom(s), 1 O-atom or S-atom, 1 N-atom and 1 O-atom or 1 N-atom and 1 S-atom, and which aryl and heteroaryl radicals are unsubstituted or substituted by lower alkyl, lower alkoxy, lower alkylthio and/or by halogen, R₂ is hydrogen, lower alkyl or lower alkenyl, and alk is lower alkylene, or a salt thereof.

4. A process according to claim 1 for the preparation of a compound of formula I wherein R₁ is a radical of formula 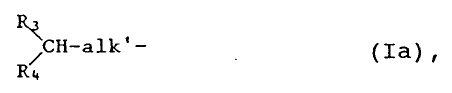 wherein R₃ is a phenyl, thienyl, pyridyl, imidazolyl or naphthyl radical that is unsubstituted or mono- or di-substituted by C₁-C₄alkyl, C₁-C₄alkoxy, C₁-C₄alkylthio and/or by halogen having an atomic number of up to and including 35, R₄ is hydrogen or, when R₃ is a phenyl radical that is unsubstituted or mono- or di-substituted by C₁-C₄alkyl, C₁-C₄alkoxy, C₁-C₄alkylthio and/or by halogen having an atomic number of up to and including 35, R₄ is likewise a phenyl radical that is unsubstituted or mono- or di-substituted by C₁-C₄alkyl, C₁-C₄alkoxy, C₁-C₄alkylthio and/or by halogen having an atomic number of up to and including 35, alk' is C₁-C₅alkylene, R₂ is hydrogen, and alk is straight-chained C₂-C₄alkylene, or a salt thereof.

5. A process according to claim 1 for the preparation Of a compound of formula I wherein R₁ is a radical of formula 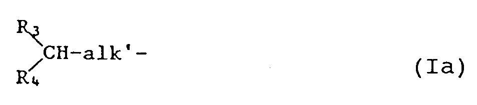 wherein R₃ is a phenyl, thienyl, pyridyl, imidazolyl or naphthyl radical that is unsubstituted or mono- or di-substituted by C₁-C₄alkyl, C₁-C₄alkoxy, C₁-C₄alkylthio and/or by halogen having an atomic number of up to and including 35, R₄ is hydrogen or, when R₃ is a phenyl radical that is unsubstituted or mono- or di-substituted by C₁-C₄alkyl, C₁-C₄alkoxy, C₁-C₄alkylthio and/or by halogen having an atomic number of up to and including 35, R₄ is likewise a phenyl radical that is unsubstituted or mono- or di-substituted by C₁-C₄alkyl, C₁-C₅alkoxy, C₁-C₄alkylthio and/or by halogen having an atomic number of up to and including 35, alk' is straight-chained C₁-C₅alkylene, R₂ is C₁-C₄alkyl or C₂-C₄alkenyl, and alk is straight-chained C₂-C₄alkylene, with the proviso that alk' is methylene and R₂ is C₁-C₂alkyl when R₃ is unsubstituted phenyl and R₄ is hydrogen, or a salt thereof.

6. A process according to claim 1 for the preparation of a compound of formula I wherein R₁ is a radical of formula 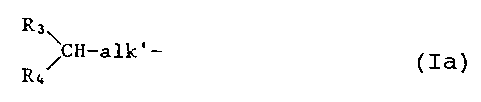 wherein R₃ is phenyl that is unsubstituted or mono- or di-substituted by C₁-C₄alkyl, C₁-C₄alkoxy and/or by halogen having an atomic number of up to and including 35, R₄ is hydrogen, alk' is straight-chained, terminally bonded C₂-C₄alkylene, R₂ is hydrogen or, when alk' is C₁-C₂alkylene, is C₁-C₃alkyl, and alk is C₂-C₃alkylene, or a salt thereof.

7. A process according to claim 1 for the preparation of a compound of formula I wherein R₁ is mono- or di-phenyl-C₂-C₆alkyl that is unsubstituted or mono- or di-substituted in the phenyl moiety by C₁-C₄alkyl, C₁-C₄alkoxy and/or by halogen having an atomic number of up to and including 35, or is imidazolyl-, thienyl- or pyridyl-C₂-C₆alkyl, R₂ is hydrogen or C₁-C₄alkyl, and alk is C₂-C₃alkylene, with the proviso that R₂ is hydrogen when R₁ is unsubstituted phenyl-C₃-C₄alkyl, or R₂ is hydrogen or C₁-C₂alkyl when R₁ is an unsubstituted phenethyl radical, or a salt thereof.

8. A process according to claim 1 for the preparation of a compound of formula I wherein R₁ is a radical of formula 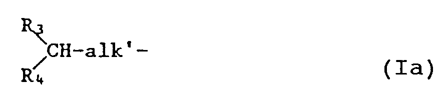 wherein R₃ is phenyl that is unsubstituted or mono- or di-substituted by C₁-C₄alkyl, C₁-C₄alkoxy and/or by halogen having an atomic number of up to and including 35, R₄ is hydrogen, alk' is straight-chained, terminally bonded C₂-C₄alkylene, R₂ is hydrogen and alk is C₂-C₃-alkylene, or a salt thereof.

9. A process for the preparation of 3-[N-(3-phenylpropyl)-N-methyl-amino]-1-hydroxy-propane-1,1-diphosphonic acid or a salt thereof.

10. A process for the preparation of 3-(3-phenylpropylamino)-1-hydroxy-propane-1,1-diphosphonic acid or a salt thereof.

11. A process for the preparation of 3-[N-(3-phenylpropyl)-N-ethyl-amino]-1-hydroxy-propane-1,1-diphosphonic acid or a salt thereof.

12. A process for the preparation of 3-[3-(pyrid-3-yl)propylamino]-1-hydroxy-propane-1,1-diphosphonic acid or a salt thereof.

13. A process for the preparation of 3-(4-phenylbutylamino)-1-hydroxy-propane-1,1-diphosphonic acid or a salt thereof.

14. A process for the preparation of 3-[4-(4-methoxyphenyl)butylamino]-1-hydroxy-propane-1,1-diphosphonic acid or a salt thereof.

15. A process for the preparation of 3-[3-(4-methoxyphenyl)propyl-N-methyl-amino]-1-hydroxy-propane-1,1- diphosphonic acid or a salt thereof.

16. A process for the preparation of 3-[3-(4-chlorophenyl)propyl-N-methyl-amino]-1-hydroxy-propane-1,1-diphosphonic acid or a salt thereof.

17. A process for the preparation of 3-[3-(3-methylphenyl)propyl-N-methyl-amino]-1-hydroxy-propane-1,1-diphosphonic acid or a salt thereof.

18. A process for the preparation of 3-[3-(pyrid-2-yl)propyl-N-methyl-amino]-1-hydroxy-propane-1,1-diphosphonic acid or a salt thereof.

19. A process for the preparation of a pharmaceutical composition which comprises mixing a compound obtainable according to any one of claims 1, 3, 6 and 8 to 12 with customary pharmaceutical carrier(s).

20. A process for the preparation of a pharmaceutical composition which comprises mixing a compound obtainable according to any one of claims 2, 4, 5, 7 and 13 to 18 with customary pharmaceutical carrier(s).

## Revendications (Revendications pour l'(les) Etat(s) contractant(s) suivant(s): AT, BE, CH, DE, FR, GB, IT, LI, LU, NL, SE)

1. Acides alkylaminoalcane-diphosphoniques à substituants aromatiques, de formule 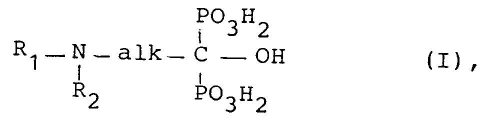 dans laquelle R₁ représente un groupe alkyle inférieur ou alcényle inférieur mono- ou di-substitué par un radical aryle monocyclique à 6 chaînons ou bicyclique constitué de cycles à 5 ou 6 chaînons ou un radical hétéroaryle contenant en tant qu'hétéroatomes 1 ou 2 atomes d'azote, 1 atome d'oxygène ou de soufre, 1 atome d'azote et 1 atome d'oxygène ou 1 atome d'azote et 1 atome de soufre, non substitué ou substitué par des groupes alkyle inférieurs, alcoxy inférieurs, alkylthio inférieurs et/ou des halogènes, R₂ représente l'hydrogène, un groupe alkyle inférieur ou alcényle inférieur et alk représente un groupe alkylène inférieur, sous réserve que R₂ représente l'hydrogène ou un groupe alkyle en C 1-C 3 et R₁ contient dans sa partie aliphatique 2 ou 3 atomes de carbone lorsque R₁ est monosubstitué par un groupe phényle non substitué, et leurs sels, les groupes et radicaux qualifiés d'"inférieurs" contenant au maximum 7 atomes de carbone.

2. Composés selon revendication 1, de formule I dans laquelle R₁ représente un groupe alkyle inférieur mono- ou di-substitué par un radical aryle monocyclique à 6 chaînons ou bicyclique constitué de cycles à 5 ou 6 chaînons non substitué ou substitué par des groupes alkyle inférieurs, alcoxy inférieurs, alkylthio inférieurs et/ou des halogènes, ou monosubstitué par un radical hétéroaryle monocyclique à 5 ou 6 chaînons contenant en tant qu'hétéroatomes 1 atome d'oxygène ou de soufre ou 1 ou 2 atomes d'azote, R₂ représente l'hydrogène ou un groupe alkyle inférieur et alk un groupe alkylène inférieur, sous réserve que R₂ représente l'hydrogène lorsque R₁ représente un groupe benzyle ou phényl-alkyle en C 3-C 7 non substitué, et R₂ représente l'hydrogène ou un groupe alkyle inférieur en C 1 ou C 2 lorsque R₁ représente un groupe phényléthyle, et leurs sels.

3. Composés selon revendication 1, de formule I dans laquelle R₁ représente un groupe de formule 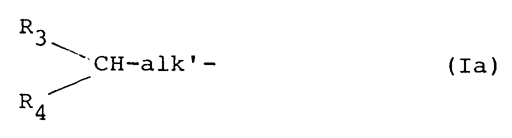 dans laquelle R₃ représente un radical aromatique R_{A}, R₄ représente l'hydrogène ou un radical aromatique R_{B} et alk' représente un groupe alkylène inférieur, R_{A} et R_{B} représentant chacun un radical aryle monocyclique à 5 ou 6 chaînons ou bicyclique constitué de cycles à 5 ou 6 chaînons ou respec tivement un radical hétéroaryle contenant en tant qu'hétéroatomes 1 ou 2 atomes d'azote, 1 atome d'oxygène ou de soufre, 1 atome d'azote et 1 atome d'oxygène ou 1 atome d'azote et 1 atome de soufre, non substitué ou substitué par des groupes alkyle inférieurs, alcoxy inférieurs, alkylthio inférieurs et/ou des halogènes, et alk représente un groupe alkylène inférieur, et leurs sels.

4. Composés selon revendication 1, de formule I dans laquelle R₁ représente un groupe de formule 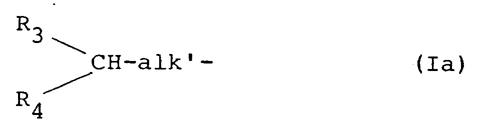 dans laquelle R₃ représente un groupe phényle, thiényle, pyridyle, imidazolyle ou naphtyle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, R₄ représente l'hydrogène ou bien encore, lorsque R₃ représente un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, alk' représente un groupe alkylène en C 1-C 5, R₂ représente l'hydrogène et alk un groupe alkylène à chaîne droite en C 2-C 4, et leurs sels.

5. Composés selon revendication 1, de formule I dans laquelle R₁ représente un groupe de formule 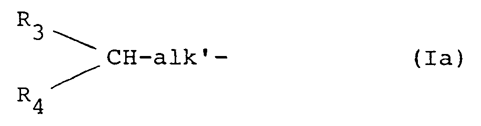 dans laquelle R₃ représente un groupe phényle, thiényle, pyridyle, imidazolyle ou naphtyle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, R₄ représente l'hydrogène ou bien encore, lorsque R₃ représente un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, un groupe phényle non substitué ou mono- ou di-substitué par des groupes alkyle en C 1-C 4, alcoxy en C 1-C 5, alkylthio en C 1-C 4 et/ou des halogènes de numéro atomique allant jusqu'à 35 inclus, alk' représente un groupe alkylène à chaîne droite en C 1-C 5, R₂ représente un groupe alkyle en C 1-C 4 ou alcényle en C 2-C 4 et alk représente un groupe alkyle à chaîne droite en C 2-C 4, sous réserve que alk' représente un groupe méthylène et R₂ un groupe alkyle en C 1-C 2 lorsque R₃ représente un groupe phényle non substitué et R₄ l'hydrogène, et leurs sels.

6. Composés selon revendication 1, de formule I dans laquelle R₁ représente un groupe de formule 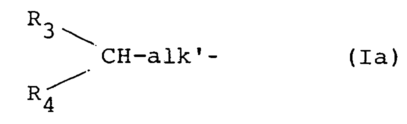 dans laquelle R₃ représente un groupe phényle non substitué ou mono- ou di-substitué par des groupes alkyle en C 1-C 4, alcoxy en C 1-C 4 et/ou des halogènes de numéro atomique allant jusqu'à 35 inclus, R₄ représente l'hydrogène, alk' représente un groupe alkylène à chaîne droite en C 2-C 4 fixé en position terminale, R₂ représente l'hydrogène ou bien encore, lorsque alk' représente un groupe alkylène en C 1-C 2, un groupe alkyle en C 1-C 3, et alk représente un groupe alkylène en C 2-C 3, et leurs sels.

7. Composés selon revendication 1, de formule I dans laquelle R₁ représente un groupe mono- ou di-phénylalkyle en C 2-C 6 ou imidazolyl-, thiényl- ou pyridylalkyle en C 2-C 6 non substitué ou mono- ou di-substitué dans la partie phényle par des groupes alkyle en C 1-C 4, alcoxy en C 1-C 4 et/ou des halogènes de numéro atomique allant jusqu'à 35 inclus, R₂ représente l'hydrogène ou un groupe alkyle en C 1-C 4 et alk représente un groupe alkylène en C 2-C 3, sous réserve que R₂ représente l'hydrogène lorsque R₁ représente un groupe phényl-alkyle en C 3-C 4 non substitué ou bien l'hydrogène ou un groupe alkyle en C 1-C 2 lorsque R₁ représente un groupe phényléthyle non substitué, et leurs sels.

8. Composés selon revendication 1, de formule I dans laquelle R₁ représente un groupe de formule 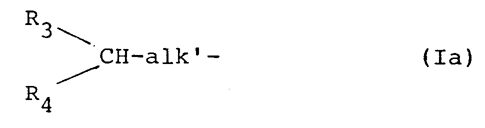 dans laquelle R₃ représente un groupe phényle non substitué ou mono- ou di-substitué par des groupes alkyle en C 1-C 4, alcoxy en C 1-C 4 et/ou des halogènes de numéro atomique allant jusqu'à 35 inclus, R₄ représente l'hydrogène, alk' un groupe alkylène à chaîne droite en C 2-C 4 fixé en position terminale, R₂ l'hydrogène et alk un groupe alkylène en C 2-C 3, et leurs sels.

9. L'acide 3-[N-(3-phénylpropyl)-N-méthylamino]-1-hydroxypropane-1,1-diphosphonique selon revendication 1 ou l'un de ses sels.

10. L'acide 3-(3-phénylpropylamino)-1-hydroxypropane-1,1-diphosphonique selon revendication 1 ou l'un de ses sels.

11. L'acide 3-[N-(3-phénylpropyl)-N-éthylamino]-1-hydroxypropane-1,1-diphosphonique selon revendication 1 ou l'un de ses sels.

12. L'acide 3-[3-(pyrido-3-yl)-propylamino]-1-hydroxypropane-1,1-diphosphonique selon revendication 1 ou l'un de ses sels.

13. L'acide 3-(4-phénylbutylamino)-1-hydroxypropane-1,1-diphosphonique selon revendication 1 ou l'un de ses sels.

14. L'acide 3-[4-(4-méthoxyphényl)-butylamino]-1-hydroxypropane-1,1-diphosphonique selon revendication 1 ou l'un de ses sels.

15. L'acide 3-[3-(4-méthoxyphényl)-propyl-N-méthylamino]-1-hydroxypropane-1,1-diphosphonique selon revendication 1 ou l'un de ses sels.

16. L'acide 3-[3-(4-chlorophényl)-propyl-N-méthylamino]-1-hydroxypropane-1,1-diphosphonique selon revendication 1 ou l'un de ses sels.

17. L'acide 3-[3-(3-méthylphényl)-propyl-N-méthylamino]-1-hydroxypropane-1,1-diphosphonique selon revendication 1 ou l'un de ses sels.

18. L'acide 3-[3-(pyrido-2-yl)-propyl-N-méthylamino]-1-hydroxypropane-1,1-diphosphonique selon revendication 1 ou l'un de ses sels.

19. Un composé selon l'une des revendications 1, 3, 6 et 8 à 12, à l'état libre ou à l'état de sel acceptable pour l'usage pharmaceutique, pour l'utilisation dans un procédé pour le traitement thérapeutique de l'organisme humain ou animal.

20. Un composé selon l'une des revendications 2, 4, 5, 7 et 13 à 18, à l'état libre ou à l'état de sel acceptable pour l'usage pharmaceutique, pour l'utilisation dans un procédé pour le traitement thérapeutique de l'organisme humain ou animal.

21. Compositions pharmaceutiques contenant un composé selon l'une des revendications 1, 3, 6, 8 à 12 et 19, avec des véhicules pharmaceutiques usuels.

22. Compositions pharmaceutiques contenant un composé selon l'une des revendications 2, 4, 5, 7, 13 à 18 et 20, avec des véhicules pharmaceutiques usuels.

23. Procédé de préparation des acides alkylaminoalcane-diphosphoniques à substituants aromatiques selon revendication 1, ou de leurs sels, caractérisé en ce que
a) dans un composé de formule 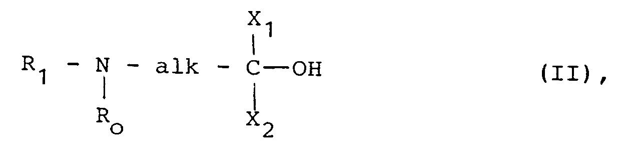 dans laquelle R₀ représente un groupe R₂ ou un groupe protecteur du groupe amino et X₁ représente un groupe phosphono ayant subi une modification fonctionnelle et X₂ un groupe phosphono libre ou ayant subi une modification fonctionnelle, on convertit le groupe phosphono X₁ ayant subi une modification fonctionnelle et le cas échéant le groupe X₂ en un groupe phosphono libre, ou bien
b) on fait réagir entre eux des composés de formules 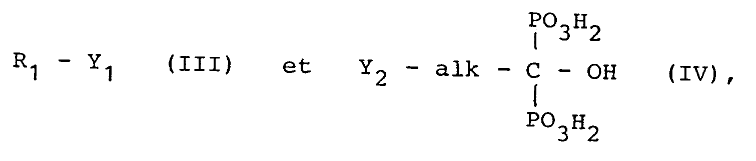 dans lesquelles l'un des symboles Y₁ et Y₂ représente un groupe hydroxy estérifié réactif et l'autre un groupe de formule -N(R₀)-H dans laquelle R₀ représente un groupe R₂ ou un groupe protecteur du groupe amino, ou des sels de ces composés, ou bien
c) on fait réagir un composé de formule 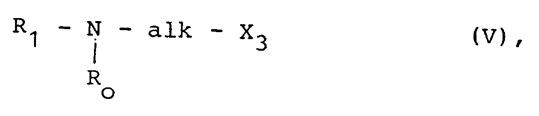 dans laquelle R₀ représente un groupe R₂ ou un groupe protecteur du groupe amino et X₃ représente un groupe carboxy, carbamyle ou cyano, plus spécialement un groupe carboxy ou cyano, avec l'acide phosphoreux et le trichlorure de phosphore, on hydrolyse ensuite le produit obtenu en premier et, dans un produit intermédiaire obtenu à partir de composés de formule V dans laquelle X₃ représente un groupe cyano ou carbamyle, et répondant à la formule 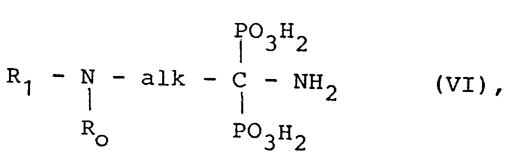 ou un sel d'un tel composé, on remplace le groupe amino par un groupe hydroxy en traitant par l'acide nitreux; dans tous les cas on élimine le groupe protecteur du groupe amino éventuellement présent et si on le désire, on convertit un composé ainsi obtenu en un autre composé de formule I et/ou un composé obtenu à l'état libre en un sel ou un sel obtenu en le composé libre ou en un autre sel.

24. Utilisation d'un composé selon l'une des revendications 1 à 20 pour la préparation d'un médicament approprié à l'utilisation pour le traitement des troubles du métabolisme du calcium et des maladies en relation avec ces troubles.

## Revendications (Revendications pour l'(les) Etat(s) contractant(s) suivant(s): ES, GR)

1. Procédé de préparation d'acides alkylaminoalcane-diphosphoniques à substituants aromatiques, de formule 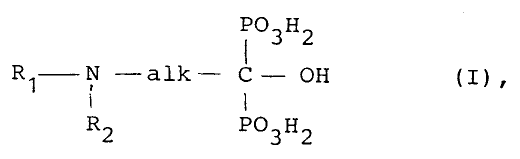 dans laquelle R₁ représente un groupe alkyle inférieur ou alcényle inférieur mono- ou di-substitué par un radical aryle monocyclique à 6 chaînons ou bicyclique constitué de cycles à 5 ou 6 chaînons ou un radical hétéroaryle contenant en tant qu'hétéroatomes 1 ou 2 atomes d'azote, 1 atome d'oxygène ou de soufre, 1 atome d'azote et 1 atome d'oxygène ou 1 atome d'azote et 1 atome de soufre, non substitué ou substitué par des groupes alkyle inférieurs, alcoxy inférieurs, alkylthio inférieurs et/ou des halogènes, R₂ représente l'hydrogène, un groupe alkyle inférieur ou alcényle inférieur et alk représente un groupe alkylène inférieur, sous réserve que R₂ représente l'hydrogène ou un groupe alkyle en C 1-C 3 et R₁ contient dans sa partie aliphatique 2 ou 3 atomes de carbone lorsque R₁ est monosubstitué par un groupe phényle non substitué, les groupes et radicaux qualifiés d'"inférieurs" contenant au maximum 7 atomes de carbone, ou de leurs sels, caractérisé en ce que
a) dans un composé de formule 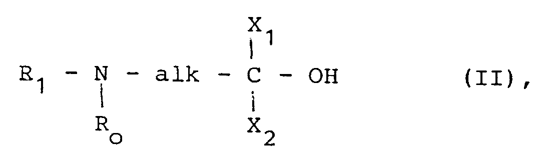 dans laquelle R₀ représente un groupe R₂ ou un groupe protecteur du groupe amino et X₁ représente un groupe phosphono ayant subi une modification fonctionnelle et X₂ un groupe phosphono libre ou ayant subi une modification fonctionnelle, on convertit le groupe phosphono X₁ ayant subi une modification fonctionnelle et le cas échéant le groupe X₂ en un groupe phosphono libre, ou bien
b) on fait réagir entre eux des composés de formule 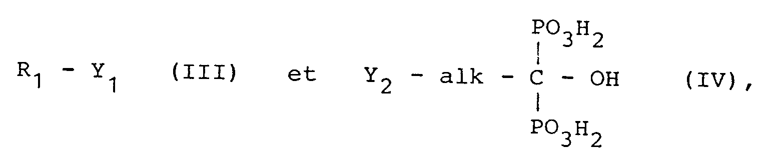 dans lesquelles l'un des symboles Y₁ et Y₂ représente un groupe hydroxy estérifié réactif et l'autre un groupe de formule -N(R₀)-H dans laquelle R₀ représente un groupe R₂ ou un groupe protecteur du groupe amino, ou des sels de ces composés, ou bien
c) on fait réagir un composé de formule 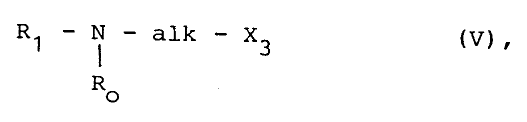 dans laquelle R₀ représente un groupe R₂ ou un groupe protecteur du groupe amino et X₃ représente un groupe carboxy, carbamyle ou cyano, plus spécialement un groupe carboxy ou cyano, avec l'acide phosphoreux et le trichlorure de phosphore, on hydrolyse ensuite le produit obtenu en premier et, dans un produit intermédiaire obtenu à partir de composés de formule V dans laquelle X₃ représente un groupe cyano ou carbamyle, et répondant à la formule 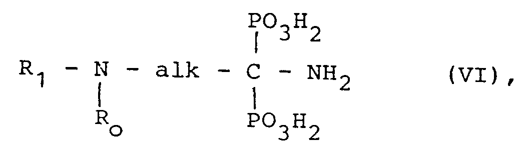 ou un sel d'un tel composé, on remplace le groupe amino par un groupe hydroxy en traitant par l'acide nitreux; dans tous les cas on élimine le groupe protecteur du groupe amino éventuellement présent et si on le désire, on convertit un composé ainsi obtenu en un autre composé de formule I et/ou un composé obtenu à l'état libre en un sel ou un sel obtenu en le composé libre ou en un autre sel.

2. Procédé selon la revendication 1 pour la préparation des composés de formule I dans laquelle R₁ représente un groupe alkyle inférieur mono- ou di-substitué par un radical aryle monocyclique à 6 chaînons ou bicyclique constitué de cycles à 5 ou 6 chaînons non substitué ou substitué par des groupes alkyle inférieurs, alcoxy inférieurs, alkylthio inférieurs et/ou des halogènes, ou monosubstitué par un radical hétéroaryle monocyclique à 5 ou 6 chaînons contenant en tant qu'hétéroatomes 1 atome d'oxygène ou de soufre ou 1 ou 2 atomes d'azote, R₂ représente l'hydrogène ou un groupe alkyle inférieur et alk un groupe alkylène inférieur, sous réserve que R₂ représente l'hydrogène lorsque R₁ représente un groupe benzyle ou phényl-alkyle en C 3-C 7 non substitué, et R₂ représente l'hydrogène ou un groupe alkyle inférieur en C 1 ou C 2 lorsque R₁ représente un groupe phényléthyle, et leurs sels.

3. Procédé selon la revendication 1, pour la préparation des composés de formule I dans laquelle R₁ représente un groupe de formule 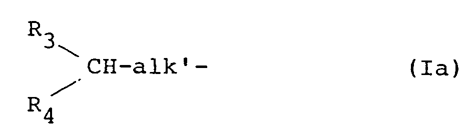 dans laquelle R₃ représente un radical aromatique R_{A}, R₄ représente l'hydrogène ou un radical aromatique R_{B} et alk' représente un groupe alkylène inférieur, R_{A} et R_{B} représentant chacun un radical aryle monocyclique à 5 ou 6 chaînons ou bicyclique constitué de cycles à 5 ou 6 chaînons ou respectivement un radical hétéroaryle contenant en tant qu'hétéroatomes 1 ou 2 atomes d'azote, 1 atome d'oxygène ou de soufre, 1 atome d'azote et 1 atome d'oxygène ou 1 atome d'azote et 1 atome de soufre, non substitué ou substitué par des groupes alkyle inférieurs, alcoxy inférieurs, alkylthio inférieurs et/ou des halogènes, et alk représente un groupe alkylène inférieur, et leurs sels.

4. Procédé selon revendication 1, pour la préparation des composés de formule I dans laquelle R₁ représente un groupe de formule 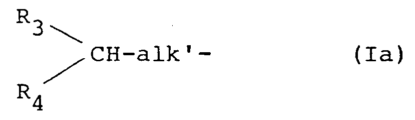 dans laquelle R₃ représente un groupe phényle, thiényle, pyridyle, imidazolyle ou naphtyle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, R₄ représente l'hydrogène ou bien encore, lorsque R₃ représente un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, alk' représente un groupe alkylène en C 1-C 5, R₂ représente l'hydrogène et alk un groupe alkylène à chaîne droite en C 2-C 4, et leurs sels.

5. Procédé selon revendication 1, pour la préparation des composés de formule I dans laquelle R₁ représente un groupe de formule 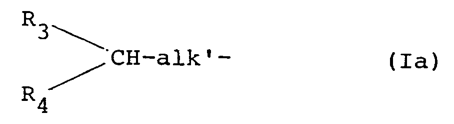 dans laquelle R₃ représente un groupe phényle, thiényle, pyridyle, imidazolyle ou naphtyle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, R₄ représente l'hydrogène ou bien encore, lorsque R₃ représente un groupe phényle non substitué ou mono- ou di-substitué par des groupes alkyle en C 1-C 4, alcoxy en C 1-C 4, alkylthio en C 1-C 4 et/ou des halogènes de numéro atomique allant jusqu'à 35 inclus, un groupe phényle non substitué ou mono- ou di-substitué par des groupes alkyle en C 1-C 4, alcoxy en C 1-C 5, alkylthio en C 1-C 4 et/ou des halogènes de numéro atomique allant jusqu'à 35 inclus, alk' représente un groupe alkylène à chaîne droite en C 1-C 5, R₂ représente un groupe alkyle en C 1-C 4 ou alcényle en C 2-C 4 et alk représente un groupe alkyle à chaîne droite en C 2-C 4, sous réserve que alk' représente un groupe méthylène et R₂ un groupe alkyle en C 1-C 2 lorsque R₃ représente un groupe phényle non substitué et R₄ l'hydrogène, et leurs sels.

6. Procédé selon revendication 1, pour la préparation des composés de formule I dans laquelle R₁ représente un groupe de formule 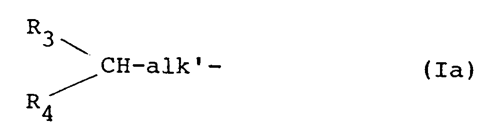 dans laquelle R₃ représente un groupe phényle non substitué ou mono- ou di-substitué par des groupes alkyle en C 1-C 4, alcoxy en C 1-C 4 et/ou des halogènes de numéro atomique allant jusqu'à 35 inclus, R₄ représente l'hydrogène, alk' représente un groupe alkylène à chaîne droite en C 2-C 4 fixé en position terminale, R₂ représente l'hydrogène ou bien encore, lorsque alk' représente un groupe alkylène en C 1-C 2, un groupe alkyle en C 1-C 3, et alk représente un groupe alkylène en C 2-C 3, et leurs sels.

7. Procédé selon revendication 1, pour la préparation des composés de formule I dans laquelle R₁ représente un groupe mono- ou di-phényl-alkyle en C 2-C 6 ou imidazolyl-, thiényl- ou pyridyl-alkyle en C 2-C 6 non substitué ou mono- ou di-substitué dans la partie phényle par des groupes alkyle en C 1-C 4, alcoxy en C 1-C 4 et/ou des halogènes de numéro atomique allant jusqu'à 35 inclus, R₂ représente l'hydrogène ou un groupe alkyle en C 1-C 4 et alk représente un groupe alkylène en C 2-C 3, sous réserve que R₂ représente l'hydrogène lorsque R₁ représente un groupe phényl-alkyle en C 3-C 4 non substitué ou bien l'hydrogène ou -un groupe alkyle en C 1-C 2 lorsque R₁ représente un groupe phényléthyle non substitué, et leurs sels.

8. Procédé selon revendication 1, pour la préparation des composés de formule I dans laquelle R₁ représente un groupe de formule 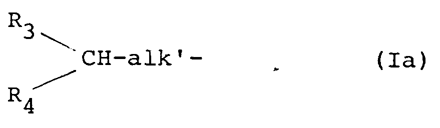 dans laquelle R₃ représente un groupe phényle non substitué ou mono- ou di-substitué par des groupes alkyle en C 1-C 4, alcoxy en C 1-C 4 et/ou des halogènes de numéro atomique allant jusqu'à 35 inclus, R₄ représente l'hydrogène, alk' un groupe alkylène à chaîne droite en C 2-C 4 fixé en position terminale, R₂ l'hydrogène et alk un groupe alkylène en C 2-C 3, et leurs sels.

9. Procédé de préparation de l'acide 3-[N-(3-phénylpropyl)-N-méthylamino]-1-hydroxypropane-1,1-diphosphonique ou de l'un de ses sels.

10. Procédé de préparation de l'acide 3-(3-phénylpropylamino)-1-hydroxy-propane-1,1-diphosphonique ou de l'un de ses sels.

11. Procédé de préparation de l'acide 3-[N-(3-phénylpropyl)-N-éthylamino]-1-hydroxypropane-1,1-diphosphonique ou de l'un de ses sels.

12. Procédé de préparation de l'acide 3-[3-(pyrido-3-yl)-propylamino]-1-hydroxypropane-1,1-diphosphonique ou de l'un de ses sels.

13. Procédé de préparation de l'acide 3-(4-phénylbutylamino)-1-hydroxypropane-1,1-diphosphonique ou de l'un de ses sels.

14. Procédé de préparation de l'acide 3-[4-(4-méthoxyphényl)-butylamino]-1-hydroxypropane-1,1-diphosphonique ou de l'un de ses sels.

15. Procédé de préparation de l'acide 3-[3-(4-méthoxyphényl)-propyl-N-méthylamino]-1-hydroxypropane-1,1-diphosphonique ou de l'un de ses sels.

16. Procédé de préparation de l'acide 3-[3-(4-chlorophényl)-propyl-N-méthylamino]-1-hydroxypropane-1,1-diphosphonique ou de l'un de ses sels.

17. Procédé de préparation de l'acide 3-[3-(3-méthylphényl)-propyl-N-méthylamino]-1-hydroxypropane-1,1-diphosphonique ou de l'un de ses sels.

18. Procédé de préparation de l'acide 3-[3-(pyrido-2-yl)-propyl-N-méthylamino]-1-hydroxypropane-1,1-diphosphonique ou de l'un de ses sels.

19. Procédé de préparation de compositions pharmaceutiques, caractérisé en ce que l'on mélange un composé obtenu selon l'une des revendications 1, 3, 6 et 8 à 12, avec des véhicules pharmaceutiques usuels.

20. Procédé de préparation de compositions pharmaceutiques, caractérisé en ce que l'on mélange un composé obtenu selon l'une des revendications 2, 4, 5, 7, et 13 à 18, avec des véhicules pharmaceutiques usuels.
